# EUROPEAN PATENT SPECIFICATION

(11) **EP 2 947 111 B1**
(45) Date of publication and mention of the grant of the patent: **07.03.2018**
(21) Application number: 13871951.3
(22) Date of filing: 29.03.2013
(51) Int. Cl.: C08G 65/32, C08G 65/28, C08G 65/329, A61K 47/50, A61K 47/34, C08G 65/26, C08G 65/331, C08G 65/08

(54) **MONOFUNCTIONAL BRANCHED POLYETHYLENEGLYCOL AND BIO-RELATED SUBSTANCE MODIFIED BY SAME**
MONOFUNKTIONELLES VERZWEIGTES POLYETHYLENGLYCOL UND DAMIT MODIFIZIERTE BIOBEZOGENE SUBSTANZ
POLYÉTHYLÈNEGLYCOL RAMIFIÉ MONOFONCTIONNEL ET SUBSTANCE DE TYPE BIOLOGIQUE MODIFIÉE PAR CELUI-CI

(30) Priority: 17.01.2013 CN 201310020124; 17.01.2013 CN 201310017350
(43) Date of publication of application: 25.11.2015
(73) Proprietor: Xiamen Sinopeg Biotech Co., Ltd., Xiamen, Fujian 361100 (CN)
(72) Inventor: WENG, Wengui, Xiamen Fujian 361100 (CN); LIU, Chao, Xiamen Fujian 361100 (CN); LIAO, Jincheng, Xiamen Fujian 361100 (CN); YUAN, Jinchun, Xiamen Fujian 361100 (CN)
(74) Representative: Hoffmann Eitle
(86) International application number: PCT/CN2013/073463
(87) International publication number: WO 2014/110867

(56) References cited:
- WO-A2-03/040211
- WO-A2-2005/000360
- CN-A- 101 010 105
- CN-A- 101 448 525
- CN-A- 101 616 678
- CN-A- 101 831 065
- CN-A- 102 367 290
- US-A- 5 919 455
- US-A1- 2009 117 077

## Description

### TECHNICAL FIELD

The present invention relates to polyethylene glycol derivatives. In particular, the invention relates to a monofunctional branched polyethylene glycol (PEG) and a bio-related substance modified with the monofunctional branched polyethylene glycol.

### BACKGROUND ART

PEGylation has been widely recognized as one of the most important approaches for drug modification. Functionalized PEGs, owing to their active groups, are capable of modifying therapeutic drugs and bio-related substance by covalently binding to target molecules, normally small molecule organic drugs or biomolecules, including proteins, peptides, saccharides, lipids, oligonucleotides, affinity ligands, cofactors, liposomes, biomaterials and the like. The pegylated drugs would be endowed with many beneficial properties with respect to hydrophilicity, flexibility, antithrombogenicity, etc. Meanwhile, due to the steric repulsion effect, pharmaceutic drugs modified with polyethylene glycol can avoid the filtration through glomeruli in the kidney and the bio-reactions such as immunoreaction, so that longer half-lives in blood are achieved compared with the unmodified forms. For example, it has been shown that the water-insoluble drug paclitaxel, when coupled to polyethylene glycol, becomes water-soluble (Greenwald et al., J. Org. Chem. 1995, 331-336).

A sufficient molecular mass of polyethylene glycol is needed in order to fully improve the state of pharmaceuticals in vivo to obtain increased hydrophilicity, enhanced half-life and reduced immunogenicity without weakening their biological activities. However, the number of active groups in proteins and other biomolecules which can be availably modified is relatively small. As a result, the connection between the polyethylene glycol and the drug molecule to be modified becomes essentially important for getting adequate molecular mass of polyethylene glycol. Compared with a linear polyethylene glycol having the same molecular weight, a branched polyethylene glycol, in virtue of its particular spatial structure, can provide an "umbrella-like" protective coverage around protein surface which increases steric hindrance around the drug molecules. Such a branched structure may inhibit attack from other macromolecules in vivo more effectively to further decrease inactivation and enzymolysis in body, and therefore enable a more prolonged circulation time of the corresponding pegylated drugs.

In 1995, Monfardini and coworkers synthesized a branched polyethylene glycol with two arms, also denoted as "V-shape" PEG, wherein two linear monomethoxy polyethylene glycol chains were directly linked to the two amino groups of lysine followed by activation of the carboxyl group as succinimidyl ester, and furthermore modification of enzymes with the branched polyethylene glycol was investigated (Bioconjugate Chem. 1995, 6, 62-69). Since then, it has gained popularity as a method to produce a monofunctional branched PEG as well as derivatives thereof, and has already been applied in three commercially available pharmaceutical products. Nevertheless, this technology suffers from some drawbacks such as low reaction efficiency, long synthesis period and instability under basic conditions. Besides, the asymmetry between the two amino groups of lysine will cause inhomogenecity during the modification process to result in the formation of monomodified by-products or the addition of polyethylene glycol in great excess. As a result, the difficulty and cost of purification are increased.

Furthermore, in the case of pegylated interferon α conjugates, interferon α links to functional polyethylene glycol via three urethane and amide bonds, but these bonds are labile to hydrolysis during the reaction under a basic condition or during storage which may affect the efficacy and usage of drugs.

Additionally, multi-armed star polyethylene glycol reported in the literature, which often shows good regularity and low polydisperty, can be synthesized via simultaneous initiation of small molecules having multiple active groups including 2-hydroxymethyl-1,3-propylene glycol and pentaerythritol (Macromolecules 2000, 33, 5418-5426). Gnanou et al. have prepared polyethylene glycol with a dendrimer-like structure (Polymer 2003, 44, 5067-5074). However, these multi-armed PEGs usually have the same hydroxyl group on the terminal of each arm, and hence specific reactions cannot be carried out.

US 5,919,455 defines a branched substantially non-antigenic polymer comprising a specified formula wherein the polymer is preferably prepared from polyalkylene oxids and are water-soluble at room temperature. The polymers of this document react with biologically active nucleophiles to form a conjugate. WO 2005/000360 is directed to a polymeric reagent comprising a 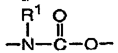 moiety positioned between a water-soluble polymer and a reactive group wherein the nitrogen atom in the above-mentioned moiety is proximal to the water-soluble polymer, the carbonyl carbon atom of this moiety is proximal to the reactive group and wherein R¹ is a hydrogen or an organic radical. The polymeric reagent is suitable in forming conjugates of amdoxivir. WO 03/040211 defines a branched reactive polymer having a specified structure which is useful for conjugation to biologically active molecules in a manner that tends to avoid a significant reduction in the biological activity of the molecules whilst still providing the benefits of water-soluble polymer conjugation.

Accordingly, it is necessary to develop a monofunctional branched polyethylene glycol which can be produced in a convenient manner and has easily controllable parameters, a process for producing the same, and a modified bio-related substance.

### DISCLOSURE OF THE INVENTION

Regarding the term of "stable" in the present invention, the conditions are limited to a physiological condition (e.g., acidic stomach, weak acidic tumor site), a mimicked physiological condition (e.g., buffer solution, culture media), or a therapeutically physiological condition (e.g., heat, ultraviolet light, visible light, infrared light, near infrared light, mid-infrared light), since the monofunctional branched poly(ethylene glycol) in the present invention is reactive with a "physiologically active bio-substance or a modified derivative thereof' via functional group "R" which is capable of reacting with the reactive group of a bio-related substance. Herein, "stable" is relatively compared with "degradable". In the present invention, an urethane group as well as an ester group, a thioester group, a disulfide group, a carbonate group and etc are herein considered to be an "unstable" or a "degradable" group, particularly during metabolic processes in vivo. Vividly, if the PEG-drug conjugates keeps as a whole during the circulation time, the linker can be considered as a "stable", "permanent", "undegradable" linkage. On the contrary, the linkage can be described as "unstable", "releasable", "degradable, e.g. hydrolytically or enzymatically degradable", "cuttable", "cleavable", "reversible" etc. The applicant believes that the persons skilled in the art can understand the meaning of "stable" through the above explanation.

The purpose of this invention is to overcome the shortcomings of the prior art and to provide a monofunctional branched polyethylene glycol. Such a monofunctional branched polyethylene glycol can get over the defects of the traditional multi-armed PEGs in the application of drug modification. The bio-related substance can be modified under relatively mild conditions. There still exist some other advantages, e.g., the ratio of functionalization is high, the amount of by-product is low and the activity maintainance of the modified bio-related substance is excellent.

This invention also provides a bio-related substance modified by the above-mentioned monofunctional branched polyethylene glycol, also referred to as "PEG-modified bio-related substance" in the present invention.

The above-described purposes of this invention can be realized via embodiments below.

The monofunctional branched polyethylene glycol is represented by the following general formula (1): 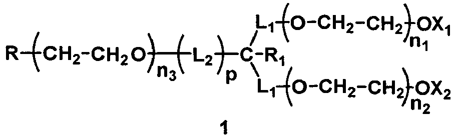 the various radicals are defined in claim 1 and wherein the monofunctional branched polyethylene glycol is obtained by the process as defined in claim 1.

The PEG-modified bio-related substance in the present invention has the following general formula (2):

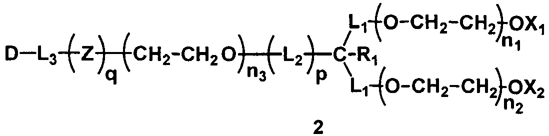

The monofunctional branched polyethylene glycol in formula (1) reacts with the reactive group of a bio-related substance via the functional group R as shown in formula (1) to form the PEG-modified bio-related substance as shown in formula (2). Wherein X₁ and X₂ are each independently a hydrocarbon group having 1 to 20 carbon atoms at the terminal end of the two branch chains, n₁ and n₂ are each independently a value selected from 1 to 1000, n₃ is a value selected from 11 to 1000, L₁ and L₂ are each independently a linking group which is stable under the conditions of light illumination, enzyme, acid or base, p is 0 or 1, and q is 0 or 1, R₁ is a hydrogen atom or a hydrocarbon group having 1 to 20 carbon atoms, D is a bio-related substance, Z is a linking group through which a functional group capable of reacting with the bio-related substance is linked onto the main chain of the branched polyethylene glycol, and can react with the bio-related substance to form a residue group L₃.

The present invention also provides a production process of the monofunctional branched polyethylene glycol including five steps as follows:
Step (a): In a coinitiator system consisting of a small molecule initiator (4) and a base, ethylene oxide is polymerized to the two geometrically symmetrical hydroxyl groups of initiator (4) to generate two branch chains. Thereafter, the terminal ends of the two newly formed branch chains are deprotonated to obtain an intermediate (5).
Step (b): The deprotonated two branch chains of intermediate (5) are alkyl-etherified to obtain an intermediate (6). "Alkyl-etherified" is also referred to as "end-capped", while "alkyl-etherification" is also referred to as "end-capping".
Step c): The terminal hydroxyl group on the symmetry axis of intermediate (6) is deprotected to obtain an intermediate (7).
Step (d): Ethylene oxide is polymerized to the deprotected terminal hydroxyl group on the symmetry axis of intermediate (7) to generate a main chain, which is subsequently protonated to obtain an intermediate (3) with a terminal hydroxyl group.
Step (e): The terminal of the main chain of intermediate (3) is functionalized, and thereby the monofunctional branched polyethylene glycol of the general formula (1) can be obtained. 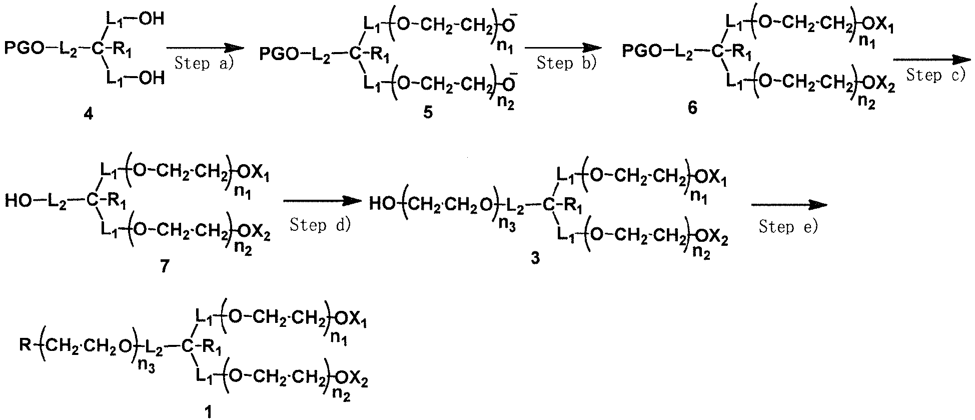
wherein PG represents a protective group of a hydroxyl group, PG can be silyl-ether, benzyl, acetal, ketal or tertiary butyl, and the definitions of X₁, X₂, n₁, n₂, n₃, L₁, L₂, p and R₁ are the same as those in general formula (1).

The monofunctional branched polyethylene glycol can be applied in the modification of bio-related substances.

The present invention afford some advantages over the prior art which are explained in the following.

With regard to the monofunctional branched polyethylene glycol of the present invention, the active functional group is disposed at the terminal end of the main chain. Moreover, steric hindrance owing to the polyethylene glycol chains is small as compared with the case of conventional polyethylene glycol derivatives. As a result, the functionalization of PEG and the modification to the bio-related substance can be conveniently conducted under much milder conditions. Meanwhile, the ratio of functionalization increases, the amount of by-products decreases, and the activity maintainance of the modified bio-related substance gets better. Furthermore, in the production method of the present invention, the two hydroxyl groups of the small molecule initiator having a geometrically symmetrical structure possess the same reactivity, and thus have close rates of chain propagation to obtain two branch chains having close or identical degree of polymerization. What's more, the inhomogenecity between the two branch chains decreases, the repreatability of modification to the bio-related substance increases, and the activity of the modified bio-related substance becomes more stable. Additionally, the molecular weight of the main chain and the two branch chains can be controlled in a simple and precise manner, and the structure is simply and precisely adjustable. Also, the synthesis time can be saved and the difficulty in purification can be reduced.

With regard to the bio-related substance modified with a monofunctional branched polyethylene glycol, it affords good biological activity, better solubility and a longer metabolic half-life. During its production process, the active group of the monofunctional branched polyethylene glycol is subjected to small steric hindrance. As a result, the functionalization of PEG and the modification to the bio-related substance can be conveniently carried out, and the reaction is allowed to be conducted under much milder conditions. Meanwhile, the ratio of functionalization is increased, the amount of by-products is reduced, and the activity maintainance of the modified bio-related substance is enhanced.

### DETAILED DESCRIPTION OF THE INVENTION

The present invention provides a monofunctional branched polyethylene glycol having a general formula as shown in the general formula (1): 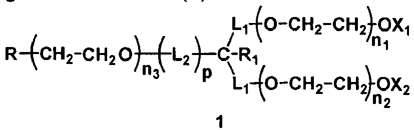 wherein the radicals are as defined in claim 1.

X₁ and X₂ can be the same or different from each other. X₁ and X₂ are preferably hydrocarbon groups having 1 to 5 carbon atoms. Preferable examples of X₁ and X₂ include hydrocarbon groups such as a methyl group, an ethyl group, a propyl group, a propenyl group, a propinyl group, an isopropyl group, a butyl group, a tertiary butyl group, a pentyl group, a heptyl group, a 2-ethylhexyl group, an octyl group, a nonyl group, a decyl group, an undecyl group, a dodecyl group, a tridecyl group, a tetradecyl group, a pentadecyl group, a hexadecyl group, a heptadecyl group, an octodecyl group, a nonadecyl group, an eicosyl group, a benzyl group or a butylphenyl group. X₁ and X₂ are most preferably a methyl group.

Preferably, R₁ is a hydrogen atom or an alkyl group having 1 to 20 carbon atoms.

Regarding R₁, the alkyl group is preferably a methyl group, an ethyl group, a 1-propyl group, an isopropyl group, a butyl group, a pentyl group, a hexyl group, a propenyl group or a benzyl group.

R is a functional group, and is preferably a functional group reactive with a bio-related substance. The bio-related substance includes the modified bio-related substance and the unmodified form. As used herein, R is selected from but not limited to the following groups:

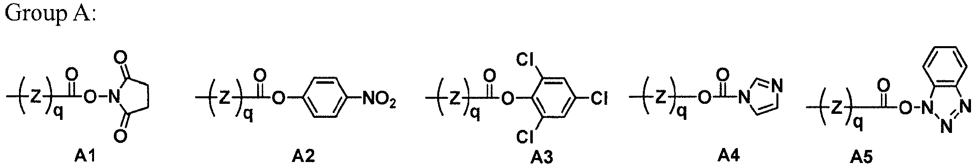

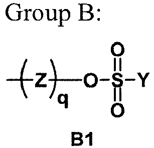

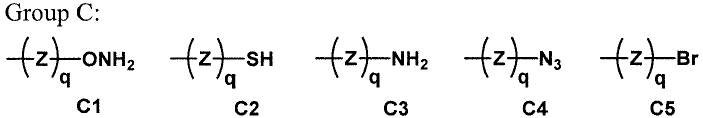

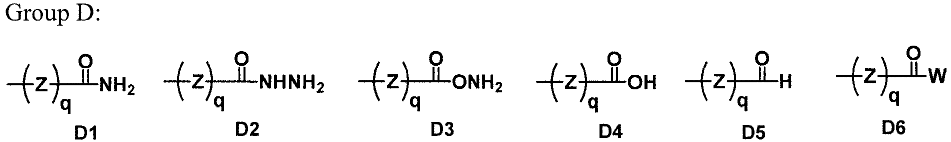

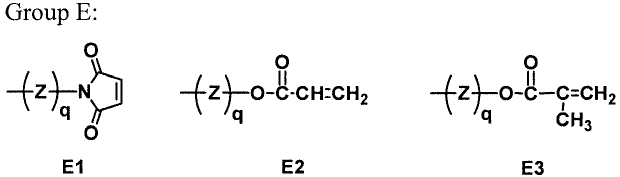

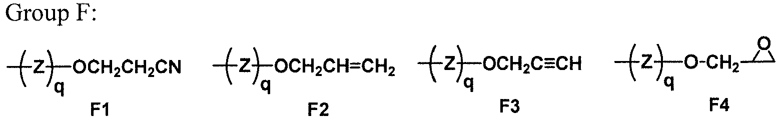

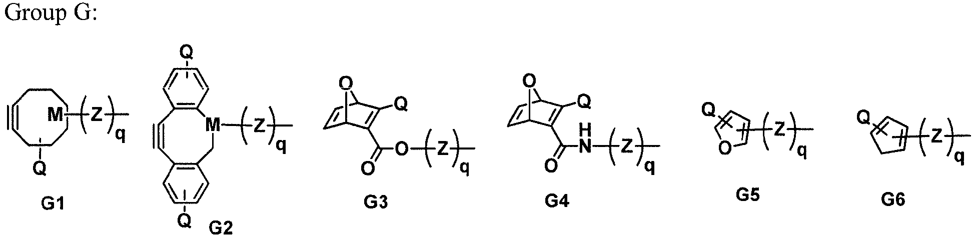

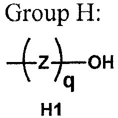

In the groups from A to H, Z is a covalent linking group between polyethylene glycol and the functional group. Z is not particularly limited. q is 0 or 1. Z can be an alkylidene group or an alkylidene group containing at least one groupselected from a group consisting of an ester bond, a urethane bond, an ether bond, a double bond, a triple bond, a carbonate bond, a secondary amino group and the like, wherein each group is stable under conditions of light illumination, enzyme, acid or base. Z is preferably an alkylidene group or an alkylidene group containing at least one group selected from a group consisting of an ether bond, an amide bond, and a secondary amino group. The alkylidene group is preferably a methylene group, a 1,2-ethylidene group, a 1,3-propylidene group, a 1,2-propylidene group, an isopropylidene group, a butylidene group, a pentylidene group or a hexylidene group.

In group B, Y is a hydrocarbon group having 1 to 10 carbon atoms or a hydrocarbon group having 1 to 10 carbon atoms which may contain fluorine atom. Y is preferably a methyl group, an ethyl group, a propyl group, an isopropyl group, a butyl group, a tertiary butyl group, a pentyl group, a hexyl group, a heptyl group, an octyl group, a nonyl group, a decyl group, a vinyl group, a phenyl group, a benzyl group, a p-methylphenyl group, a trifluoromethyl group, a 2,2,2-trifluoroethyl group or a 4-(trifluoromethoxy)phenyl group, more preferably a methyl group, a p-methylphenyl group, a 2,2,2-trifluoroethyl group, a trifluoromethyl group or a vinyl group.

In D, W represents a halogen atom, preferably Br or Cl.

In the group G, Q is not particularly limited as long as it favors inductive effect or/and conjugated effect of electrons of unsaturated bond. With respect to the ring, Q can be one or more. Q is preferably a hydrogen atom, a halogen atom, an alkyl halide group, an alkoxy group, a carbonyl compound or nitro compound, more preferably a hydrogen atom, a fluorine atom, a trifluoromethyl group or a methoxy group.

In group G, M is an atom on the ring that is linked to Z. M is a carbon atom or a nitrogen atom.

n₁ and n₂ in formula (1) represent the degree of polymerization of the two branch chains. n₁ and n₂ are each independently preferably a value from 10 to 800, more preferably from 25 to 800, most preferably from 50 to 500.

n₃ in formula (1) represents the degree of polymerization of the main chain. n₃ is preferably a value from 11 to 800, more preferably from 11 to 500, most preferably from 11 to 200.

This invention also provides a bio-related substance modified by the above-mentioned monofunctional branched polyethylene glycol which has a chemical structure as shown in the general formula (2):

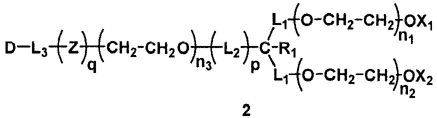

The monofunctional branched polyethylene glycol in formula (1) reacts with the reactive group of a bio-related substance via the functional group R as shown in formula (1) to form the PEG-modified bio-related substance as shown in fomula (2).

The reactive group of the bio-related substance can be an amino group, a sulphydryl group, an unsaturated bond, a carboxyl group or the like.

As used herein, L₃ is a covalent bond group linking the bio-related substance and polyethylene glycol. L₃ is the residue of the functional group on the main chain of polyethylene glycol after reacting with a bio-related substance. The linking group is not particularly limited.

L₃ can be a triazole bond, an isoxazole bond, an ether bond, an amide bond, an imide bond, an imino group, a secondary amino group, a tertiary amino group, a thioester bond, a disulfide bond, a urethane bond, a thiocarbonate bond, a sulfonate bond, a sulfamide bond, a carbamate bond, a tyrosine group, a cysteine group, a histidine group or the combination thereof.

In the general formula (2), D represents a bio-related substance including but not limited to the group consisting of polypeptide, protein, enzyme, small molecule drug, dye, liposome, nucleoside, nucleotide, oligonucleotide, polynucleotide, nucleic acid, polysaccharide, steroid, lipid, phospholipid, glycolipid, glycoprotein, cell, virus and micelle. Wherein, D is preferably selected from the group consisting of a bio-related substance and a modified bio-related substance. The small molecule drugs are not particularly limited but preferably include anticancer drugs and antifungal drugs.

Furthermore, the definitions of X₁, X₂, n₁, n₂, n₃, L₁, L₂, p, q, R₁ and Z in the general formula (2) are the same as those in the general formula (1). X₁ and X₂ are each independently a hydrocarbon group having 1 to 20 carbon atoms at the terminal end of the two branch chains, n₁ and n₂ are each independently a value selected from 1 to 1000, n₃ is a value selected from 11 to 1000, L₁ and L₂ are each independently a linking group which is stable under the conditions of light illumination, enzyme, acid or base, p and q are each independently 0 or 1, and R₁ is a hydrogen atom or a hydrocarbon group having 1 to 20 carbon atoms. Z is a covalent linking group between the main chain of PEG and residue group L₃, through which a functional group capable of reacting with the bio-related substance is linked to the main chain of polyethylene glycol. Z is not particularly limited.

The monofunctional branched polyethylene glycol in formula (1) can be produced from the intermediate compound (3) through one-step or multi-step reactions. The intermediate compound (3) is represented by 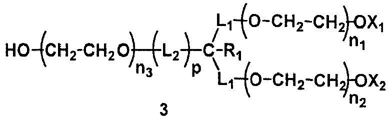 wherein the definitions of X₁, X₂, n₁, n₂, n₃, L₁, L₂, p, q and R₁ are the same as those in the general formula (1).

The present invention also provides a production process of the monofunctional branched polyethylene glycol from the intermediate (3) including the following five steps.
Step (a): In a coinitiator system consisting of a small molecule initiator (4) and a base, ethylene oxide is polymerized to the two geometrically symmetrical hydroxyl groups of initiator (4) to generate two branch chains. Thereafter, the terminal ends of the two newly formed branch chains are deprotonated to obtain an intermediate (5).
Step (b) The deprotonated two branch chains of intermediate (5) are alkyl-etherified to obtain an intermediate (6).
Step c) The terminal hydroxyl group on the symmetry axis of intermediate (6) is deprotected to obtain an intermediate (7).
Step (d) Ethylene oxide is polymerized to the deprotected terminal hydroxyl group on the symmetry axis of intermediate (7) to generate a main chain, which is subsequently protonated to obtain an intermediate (3) with a terminal hydroxyl group.
Step (e) The terminal of the main chain of intermediate (3) is functionalized, and thereby the monofunctional branched polyethylene glycol of the general formula (1) can be obtained.

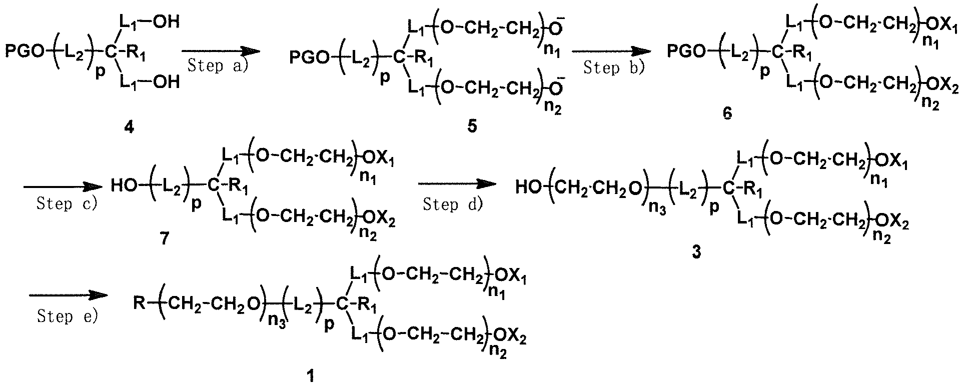

Herein, PG represents a protective group of a hydroxyl group which can be silyl-ether, benzyl, acetal, ketal or tertiary butyl. The definitions of X₁, X₂, n₁, n₂, n₃, L₁, L₂, p and R₁ are the same as those in the general formula (1). The definition of R is the same as the above-mentioned except that R equals a hydroxyl group.

### 1. Preparation of intermediate compound (3)

The preparation process of intermediate compound (3) of the present invention comprises the following steps. Firstly, ethylene oxide is polymerized, in an amount of 2 to 2000 molar to the initiator (4), to the two terminal hydroxyl groups of diol also containing a protected hydroxyl group at the terminal end of the main chain, and then excess deprotonation reagent is added to obtain a anionic PEG intermediate (5) with two PEG branch chains. Secondly, the terminal oxygen anions are alkyl-etherified (also referred to as "end-capped") by hydrocarbon groups of X₁ and X₂ to obtain the intermediate (6). Thirdly, the terminal hydroxyl group on the main chain is deprotected. Finally, polymerization of ethylene oxide is initiated by the newly formed terminal hydroxyl group followed by an addition of proton source, and then intermediate (3) can be obtained. These steps correspond to above-described steps (a) to (d).

### 1.1 Preparation of anionic PEG intermediate (5) (step (a))

The preparation method of intermediate (5) consists of two steps: polymerization of ethylene oxide using small molecule initiator and deprotonation of the resulting polymerization product.

The polymerization of ethylene oxide using small molecule initiator can be achieved via the following two steps (A) and (B). Step (A): compound (4) is deprotonated via base catalysis; step B: ethylene oxide is polymerized. These two steps can be carried out in a solvent or without any solvent. The solvent is not particularly limited, but is preferably an aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

### Step A: Deprotonation of small molecule initiator

The base used for deprotonating the compound (4) is not particularly limited, but is preferably sodium metal, potassium metal, sodium hydride, potassium hydride, sodium methoxide, potassium methoxide, potassium tert-butoxide or diphenylmethyl potassium, more preferably sodium metal, potassium metal or diphenylmethyl potassium, most preferably diphenylmethyl potassium. The catalyst amount is 5 to 80% by mol. When the ratio of the catalyst to be used is less than 5% by mol, the polymerization rate is low and heat history increases to result in the formation of impurities such as a terminal vinyl ether compound formed by vinyl etherification of the terminal hydroxyl group. Under a solvent-free condition, when the catalyst amount exceeds 50% by mol, the viscosity of the reaction solution increases or the liquid solidifies, and thus the reaction becomes inhomogeneous and purification thereof tends to be difficult. In the case that toluene or tetrahydrofuran is used as solvent, the problem of viscosity increasing or liquid solidification can be solved so that the catalyst amount can be increased up to 80% by mol.

The deprotonation is commonly conducted at 10 to 50 °C, preferably 25 to 50 °C. When the temperature is lower than 10 °C, the deprotonation does not sufficiently proceed, and the base as a nucleophile reagent participates in the anionic polymerization to form a low-molecular-weight impurity having a molecular weight 0.5 time that of the target compound. There is a possibility that such an impurity may react with a bio-related substance and change the physical properties of the resulting preparation. When the temperature is higher than 50 °C, a decomposition of the protective group occurs resulting in a high-molecular-weight impurity having a molecular weight 1.5 times that of the target compound. After the high-molecular-weight impurity is alkyl-etherified in the next step, no functional group is introduced. When the modification to a drug or the like is carried out with such impurities in presence, the resulting preparation becomes inhomogeneous and hence the quality tends to be varied. Also, the requirement for a highly pure product cannot be satisfied.

The deprotonation time is preferably 10 minutes to 24 hours and varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour. When the deprotonation time is short, the deprotonation does not sufficiently proceed, and the base as a nucleophile reagent takes part in the anionic polymerization to form a low-molecular-weight impurity having a molecular weight 0.5 time that of the target compound. When the deprotonation time is longer than 24 hours, there is a possibility that a decomposition of the protective group may occur resulting in a high-molecular-weight impurity having a molecular weight 1.5 times that of the target compound which cannot satisfy the requirement for the modification of highly pure drugs.

Potassium methoxide, potassium tert-butoxide or sodium methoxide, preferably potassium methoxide is added as a catalyst in an amount of 5 to 80% by mol, and the reaction is carried out at 25 to 80 °C, preferably 50 to 60 °C. What's more, a pressure-reducing operation should be conducted in order to facilitate the exchange of protons. Potassium methoxide, potassium t-butoxide or sodium methoxide can react with ethylene oxide during the polymerization to form a mono-etherified polyethylene glycol derivative having a molecular weight 0.5 time that of the target compound. Such a polyethylene glycol derivative will be subsequently end-capped via alkyl-etherification, and convert into a bi-etherified polyethylene glycol derivative with no functional group. The deprotonation product such as methanol or t-butanol not only acts as a proton source which may quench the reaction, but also can participate in the polymerization of ethylene oxide to form the above-mentioned mono-etherified PEG. So, the reaction should be conducted at a relatively high temperature to ensure complete protonation, preferably 50 to 60 °C, and meanwhile a pressure-reducing operation is needed to remove lower alcohols.

### Sept B: polymerization of ethylene oxide

In the case that the polymerization is conducted in an aprotic solvent, the temperature is preferably 50 to 70 °C. When the temperature is lower than 50 °C, as the molecular weight gradually increases with the progress of polymerization, the viscosity of the reaction solution increases or the liquid solidifies, and hence the reaction becomes inhomogeneous and the resulting product has a broad distribution which is not suitable for the modification of highly pure drugs. When the temperature is higher than 70 °C there is a possibility that explosive polymerization or side reactions may occur such as the vinyl etherification of terminal hydroxyl group to get a terminal vinyl ether compound.

In the case that the polymerization is conducted under solvent-free conditions, the temperature is preferably 50 to 130 °C, more preferably 80 to 110 °C. When the temperature is lower than 50 °C, the polymerization rate is low and heat history increases to result in a tendency to reduce the quality of the target product. When the temperature is higher than 130 °C, side reactions tend to occur such as vinyl etherification of the terminal hydroxyl group to form a terminal vinyl ether compound. Alike, during the polymerization, as the molecular weight gradually increases, the viscosity of the reaction solution increases or the liquid solidifies, and hence the reaction becomes inhomogeneous and the distribution of the resulting product gets broad. As a result, the polymerization is preferably carried out in an aprotic solvent, preferably tetrahydrofuran or toluene.

At the moment, the resulting polymerization product is a mixture of alcohol and oxygen anions, and calls for a complete deprotonation of the terminals of the branch chains first in order to achieve a complete alkyl-etherification.

The base to be used for deprotonating the terminals of the branch chains is not particularly limited, but is preferably sodium metal, potassium metal, sodium hydride, potassium hydride, sodium methoxide, potassium methoxide, potassium tert-butoxide or diphenylmethyl potassium, more preferably sodium metal, potassium metal or diphenylmethyl potassium, most preferably diphenylmethyl potassium. Generally, the base is in an amount of 5 to 20 molar equivalents, preferably 8 to 15 molar equivalents relative to the initiator. When the amount of the base is less than 5 molar equivalents to the initiator, the terminals of the branch chains are not sufficiently deprotonated, and thus cannot be completely end-capped. Then, the active terminal hydroxyl groups of the branch chains will participate in the subsequent polymerization reaction to form an impurity having a higher molecular weight than the target compound. Therefore, the distribution of molecular weight becomes broad and a polyfunctional impurity is generated. When such impurities are present, the activity of the resulting modified drugs may be reduced or completely lost. When the base amount exceeds 20 molar equivalents to the initiator, the excess reagent tends to cause difficulty in the purification process and result in side reactions in the subsequent steps.

The deprotonation of the terminals of the branch chains is commonly carried out at 10 to 50 °C, preferably 25 to 50 °C. When the temperature is lower than 10 °C, the terminals of the branch chains are not completely deprotonated, and thus cannot be completely end-capped. Moreover, the active terminal hydroxyl groups of the branch chains will participate in the subsequent polymerization reaction to form an impurity having a higher molecular weight than the target compound. Hence, the distribution of molecular weight becomes broad, and a polyfunctional impurity is formed. When such impurities are present, the activity of the resulting modified drugs may be reduced or completely lost. When the temperature is higher than 50 °C, a decomposition of the protective group occurs, and no functional group is introduced after the resulting impurity is alkyl-etherified in the next step. When a drug or the like is modified while such impurities are present, the resulting preparation becomes inhomogeneous and hence the quality tends to be varied. Also, the preparation cannot meet the requirement for a highly pure product.

The deprotonation time is preferably 10 minutes to 24 hours and varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions. The deprotonation time of such a strong base is usually 10 minutes to 24 hours, preferably 20 minutes to 1 hour. When the deprotonation time is longer than 24 hours, there is a possibility that a decomposition of the terminal hydroxyl-protecting group on the symmetry axis may occur.

### 1.2 End-capping of the anionic PEG intermediate (5) (step b)

The end-capping (also referred to as alkyl-etherification) of the terminal of the anionic PEG intermediate (5) can be realized by either of the following (1) or (2):
(1) The anionic PEG intermediate (5) reacts with the compound (8), such as alkyl halide, alkyl sulfonate and the like, which contains a leaving group. As used herein, the compound (8) is represented as follows:

   **X-LG₁** **8**

   wherein X is a hydrocarbon group having 1 to 20 carbon atoms selected from, but not limited to, the group consisting of a methyl group, an ethyl group, a propyl group, a propenyl group, a propynyl group, an isopropyl group, a butyl group, a tert-butyl group, a pentyl group, a heptyl group, a 2-ethylhexyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a benzyl group and a tert-butylphenyl group. X is preferably a hydrocarbon group having 1 to 10 carbon atoms, most preferably a methyl group. LG₁ is a leaving group selected from, but not limited to, the group consisting of chlorine, bromine, iodine, mesylate, tosylate and 2,2,2-trifluoro-acetic acid sulfonate. LG₁ is preferably iodine. So, the compound containing a leaving group for capping the anionic PEG intermediate (5) is most preferably iodomethane.
   With regard to the compound (8) such as alkyl halide, alkyl sulfonate or the like which contains a leaving group, the amount of such a capping reagent is generally 5 to 20 molar equivalents, preferably 8 to 15 molar equivalents relative to the initiator. When the capping reagent is in an amount less than 5 molar equivalents to the initiator, the terminals of the branch chains are not completely end-capped, and the terminal oxygen anions will participate in the subsequent polymerization reaction to form an impurity having a higher molecular weight than the target compound. Therefore, the distribution of molecular weight becomes broad and a polyfunctional impurity is generated. When such impurities are contained, the activity of the resulting modified drugs may be reduced or completely lost. When the amount of the capping reagent exceeds 20 molar equivalents to the initiator, the excess reagent tends to cause difficulty in purification process, and result in side reactions in the subsequent steps.
   The temperature of the end-capping reaction is not particularly limited but preferably 25 to 50 °C.
(2) An activating reagent is added into the anionic PEG intermediate (5) to obtain corresponding polyethylene glycol sulfonate which subsequently undergoes a substitution reaction by deprotonated alcohol (X-OH) to form the compound (6). Commonly used activating reagents include methanesulfonyl chloride, p-toluenesulfonic acid, and 2,2,2-trifluoro-acetic acid sulfonyl chloride.
   Complete end-capping can be achieved by both method (1) and (2). In the case of method (1), the alkyl-etherification reaction can be conducted in the same reactor as polymerization reaction while the production method is simple and convenient in process, so method (1) is more preferable.
   The resulting product can be purified by a purification means such as extraction, recrystallization, adsorption treatment, precipitation, reverse precipitation, membrane dialysis or supercritical extraction to obtain the intermediate compound (6).

### 1.3 Deprotection of intermediate compound (6) (step c)

Since the terminal hydroxyl group on the symmetry axis can be protected in four manners by a benzyl group , a silyl-ether group, an acetal group/a ketal group or tertiary butyl group, the deprotection reaction can be carried out correspondingly in the following four ways:

### A: Deprotection of the benzyl structure

The deprotection of the benzyl structure can be achieved by hydrogenation using a hydrogenative reduction catalyst and a hydrogen donor. As used herein, the water content should be less than 1% in order to facilitate the reaction. When the water content is more than 1%, the decomposition reaction of the polyethylene glycol chain occurs. The resulting low-molecular-weight polyethylene glycol with a hydroxyl group, which can participate in the subsequent polymerization reaction or functional modification, will introduce impurities into the target product. Such impurities may even react with the bio-related substance and change the property of the preparation.

The hydrogenative reduction catalyst is preferably palladium. The carrier is not particularly limited, but is preferably alumina or carbon, more preferably carbon. The amount of palladium is 1 to 100 % by weight, preferably 1 to 20% by weight to the intermediate compound (6). When the amount of palladium is less than 1% by weight, the rate and the conversion of deprotection decrease. For the compounds that are not deprotected, subsequent polymerization or functionalization is not allowed to proceed, which will result in low ratio of functionalization of the final product. However, when the amount of palladium exceeds 100 % by weight, the polyethylene glycol chain tends to undergo a decomposition reaction.

The reaction solvent is not particularly limited as far as it allows the reagents and the product to be dissolved. Preferable solvents include methanol, ethanol, ethyl acetate, tetrahydrofuran, and more preferable is methanol. The hydrogen donor is not particularly limited, but is preferably hydrogen, cyclohexene, 2-propanol or the like. The reaction temperature is preferably 25 to 40 °C. When the temperature is higher than 40 °C, the decomposition reaction of the polyethylene glycol chain may occur. The reaction time is not particularly limited as far as it is negatively correlated with the amount of catalyst, preferably 1 to 5 hours. When the reaction time is shorter than one hour, the conversion is relatively low. When the reaction time is longer than 5 hours, the polyethylene glycol chain may undergo a decomposition reaction.

### B: Deprotection of the acetal or ketal structure

The acetal or ketal compound used for protecting such a hydroxyl group is preferably ethyl vinyl ether, tetrahydropyran, acetone, 2,2-dimethoxypropane, benzaldehyde or the like. The deprotection of the acetal or ketal structure should be carried out under an acidic condition, and the pH of the solution is preferably 0 to 4. When the pH is higher than 4, the acidity is too weak for the protective group to be completely removed. When the pH is lower than 0, the acidity is too strong so that the polyethylene glycol chain tends to undergo a decomposition reaction. The acid is not particularly limited, but is preferably acetic acid, phosphoric acid, sulfuric acid, hydrochloric acid or nitric acid, more preferably hydrochloric acid. The reaction solvent is not particularly limited as long as it allows the reagents and the product to be dissolved. The solvent is preferably water. The reaction temperature is preferably 0 to 30 °C. When the temperature is lower than 0 °C, the reaction rate is relatively slow, and the protective group cannot be completely removed. When the temperature is higher than 30 °C, the decomposition reaction of the polyethylene glycol chain tends to occur under an acidic condition.

### C: Deprotection of the siloxane structure

The compound used for protecting such a hydroxyl group is preferably trimethylsilyl ether, triethylsilyl ether, tert-butyldimethylsilyl ether, tert-butyldiphenylsilyl ether or the like. The deprotection reaction of such a siloxane structure is in need of a fluorinion-containing compound which is preferably tetrabutylammonium fluoride, tetraethylammonium fluoride, hydrofluoric acid or potassium fluoride, more preferably tetrabutylammonium fluoride or potassium fluoride. The amount of the fluorine-containing compound is 5 to 20 molar equivalents, preferably 8 to 15 molar equivalents relative to the initiator. When the amount of the fluorine-containing compound is less than 5 molar equivalents to the initiator, the deprotonation reaction cannot sufficiently proceed. When the amount exceeds 20 molar equivalents to the initiator, the excess reagent tends to cause difficulty in the purification process and result in side reactions in the subsequent steps. The reaction solvent is not particularly limited as long as it can dissolve the reagents and the product. The solvent is preferably an aprotic solvent, more preferably tetrahydrofuran or dichloromethane. The reaction temperature is preferably 0 to 30 °C. When the temperature is lower than 0 °C, the reaction rate is relatively slow, and the protective group cannot be completely removed.

### D: Deprotection of the tert-butyl structure

The deprotection of the tert-butyl structure is carried out under an acidic condition, and the pH of the solution is preferably 0 to 4. When the pH is higher than 4, the acidity is too weak to for the protective group to be completely removed. When the pH is lower than 0, the acidity is too strong, and there is a tendency for the polyethylene glycol chain to undergo a decomposition reaction. The acid is not particularly limited, but is preferably acetic acid, phosphoric acid, sulfuric acid, hydrochloric acid or nitric acid, more preferably hydrochloric acid. The reaction solvent is not particularly limited as far as it can dissolve the reagents and the product. The solvent is preferably water. The reaction temperature is preferably 0 to 30 °C. When the temperature is lower than 0 °C, the reaction rate is relatively slow, and the protective group cannot be completely removed. When the temperature is higher than 30 °C, the decomposition reaction of the polyethylene glycol chain tends to occur.

The resulting product can be purified by a purification means such as extraction, recrystallization, adsorption treatment, precipitation, reverse precipitation, membrane dialysis or supercritical extraction to obtain the intermediate compound (7).

### 1.4 Polymerization of ethylene oxide to intermediate (7) and (step d)

Similar to the polymerization reaction in 1.1, the polymerization in this step is also comprised of two steps. Step A: the terminal hydroxyl group on the symmetry axis is deprotonated via base catalysis; Step B: ethylene oxide is polymerized. These two steps can be conducted in a solvent or without any solvent. The solvent is not particularly limited, but is preferably an aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

### Step A: Deprotonation of the terminal hydroxyl group on the symmetry axis

The base used for deprotonating the terminal hydroxyl group on the symmetry axis of the intermediate compound (7) is not particularly limited, but is preferably sodium metal, potassium metal, sodium hydride, potassium hydride, sodium methoxide, potassium methoxide, potassium tert-butoxide or diphenylmethyl potassium, more preferably sodium metal, potassium metal or diphenylmethyl potassium, most preferably diphenylmethyl potassium. The catalyst amount is 5 to 80% by mol. When the amount of the catalyst to be used is less than 5% by mol, the polymerization rate is low and heat history increases to cause the formation of by-products such as a vinyl ether compound formed by vinyl etherification of the terminal hydroxyl group. Under a solvent-free condition, when the amount of the catalyst exceeds 50% by mol, the viscosity of the reaction solution increases or the liquid solidifies and thus the reaction becomes inhomogeneous and purification thereof tends to be difficult. In the case that toluene or tetrahydrofuran is used as solvent, the problem of viscosity increasing or liquid solidification can be avoided so that the catalyst amount can be increased up to 80% by mol.

The deprotonation of the terminal hydroxyl group on the symmetry axis is commonly carried out at 10 to 50 °C, preferably 25 to 50 °C. When the temperature is lower than 10°C, the deprotonation does not sufficiently proceed, and the base as a nucleophile reagent participates in the anionic polymerization to form a low-molecular-weight impurity having a molecular weight 0.5 time that of the target compound. Such an impurity may react with the bio-related substance and change the physical properties of the resulting product.

The deprotonation time of the terminal hydroxyl group on the symmetry axis is preferably 10 minutes to 24 hours and varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour. When the deprotonation time is short, the deprotonation does not sufficiently proceed, and the base as a nucleophile reagent takes part in the anionic polymerization to form a low-molecular-weight impurity having a molecular weight 0.5 time that of the target compound.

Potassium methoxide, potassium tert-butoxide or sodium methoxide, preferably potassium methoxide is added as a catalyst in an amount of 5 to 80% by mol, and the reaction is conducted at 25 to 80 °C, preferably 50 to 60 °C. Moreover, a pressure-reducing operation is carried out in order to facilitate the exchange of protons. Potassium methoxide, potassium t-butoxide or sodium methoxide can react with ethylene oxide during the polymerization to result in a mono-etherified polyethylene glycol having a molecular weight 0.5 time that of the target compound. The deprotonation product (e.g. methanol or t-butanol) not only acts as a proton source which may quench the reaction, but also can participate in the polymerization of ethylene oxide to form the above-mentioned mono-etherified polyethylene glycol. As a result, the reaction should be carried out at a relatively high temperature to ensure complete protonation, preferably 50 to 60 °C, and meanwhile a pressure-reducing operation is demanded to remove lower alcohols. Step B: Polymerization of ethylene oxide to the terminal of the symmetry axis

The polymerization reaction is carried out at 50 to 130 °C.

In the case that the polymerization is conducted in an aprotic solvent, the temperature is preferably 50 to 80 °C. When the temperature is lower than 50 °C, as the molecular weight increases gradually with the progress of the polymerization, the viscosity of the reaction solution increases or the liquid solidifies, and hence the reaction becomes inhomogeneous and the resulting product has a broad distribution which is not suitable for the modification of highly pure drugs. When the temperature is higher than 80 °C, explosive polymerization or side reactions tend to occur, such as the vinyl etherification of terminal hydroxyl group to obtain a vinyl ether compound.

In the case that the polymerization is conducted under solvent-free conditions, the temperature is preferably 80 to 100 °C. When the temperature is lower than 50 °C, the polymerization rate is low and heat history increases to result in a tendency to reduce the quality of the target product. When the temperature is higher than 130 °C, side reactions tend to occur such as the vinyl etherification of the terminal hydroxyl group to form a vinyl ether compound. Alike, during the polymerization, as the molecular weight gradually increases, the viscosity of the reaction solution goes up or the liquid solidifies, and hence the reaction becomes inhomogeneous and the distribution of the resulting product gets broad. As a result, the polymerization reaction is carried out in an aprotic solvent, preferably tetrahydrofuran or toluene.

When the polymerization proceeds to a certain degree, the intermediate compound (3) which has a main chain of a given degree of polymerization can be obtained after adding proton source. Wherein, the proton source is not particularly limited as long as it can increase the reactivity of the active hydrogen. Preferable proton source is methanol, ethanol or water.

A monofunctional branched polyethylene glycol with the general formula (1) can be obtained by modifying the intermediate compound (3) according to different demands. The preparation methods with respect to different kinds of R group are illustrated respectively.

### 2. Preparation of monofunctional branched PEG (step e)

The preparation process of the monofunctional branched PEG, except that R equals hydroxyl group, is described below in detail.

### 2.1 Preparation of monofunctional branched PEG with R selected from group A

a: The active ester compound can be achieved by reacting the intermediate compound (3) with carbonate (A11, A51), haloformate (A21, A31) or carbonyldiimidazole (A41) under a basic condition. 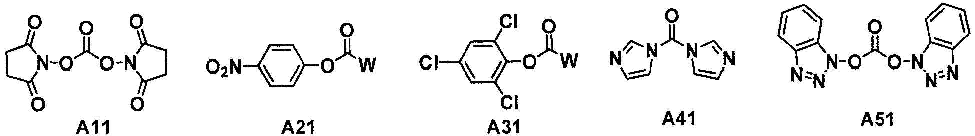 Wherein W represents Cl, Br or I, and more preferable is Cl.

The amount of carbonate (A11, A51), haloformate (A21, A31) or carbonyldiimidazole (A41) is 1 to 50 molar equivalents, preferably 1 to 20 molar equivalents, further preferably 5 to 10 molar equivalents relative to the compound (3).

The solvent can be no solvent or an aprotic solvent. The aprotic solvent can be toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide. More preferable is tetrahydrofuran, dichloromethane, dimethyl sulfoxide or dimethylformamide.

As used herein, the base to be used can be an organic base (e.g., triethylamine, pyridine, 4-dimethylaminopyridine, imidazole or diisopropylethylamine) or an inorganic base (e.g., sodium carbonate, sodium hydroxide, sodium bicarbonate, sodium acetate, potassium carbonate or potassium hydroxide). The base is preferably an organic base, more preferably triethylamine or pyridine. The amount of the base is 1 to 50 molar equivalents, preferably 1 to 10 molar equivalents, more preferably 3 to 5 molar equivalents relative to carbonate (A11, A51), haloformate (A21, A31) or carbonyldiimidazole (A41).

The reaction temperature is 0 to 200 °C, preferably 0 to 100 °C, more preferably 25 to 80 °C. The reaction time is preferably 10 mins to 48 hours, more preferably 30 mins to 24 hours. The resulting product can be purified by a purification means such as extraction, recrystallization, adsorption treatment, precipitation, reverse precipitation, membrane dialysis or supercritical extraction.
b, The ester compound can also be obtained through a condensation reaction. A carboxylic acid compound (D4) is prepared first from intermediate compound (3) through one-step or multi-step reactions, and then the carboxylic acid compound (D4) reacts with an alcohol or an amine to obtain the corresponding active ester or amide compound. 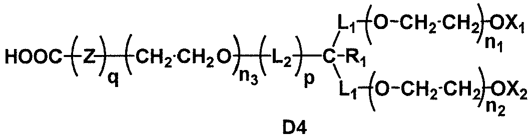 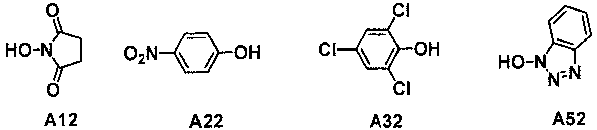 Wherein X₁, X₂, R₁, n₁, n₂, n₃, Z, L₁, L₂, p and q are the same as the above-mentioned.

The amount of N-hydroxy succinimide (A12), phenol (A22, A32) or N-hydroxy-triazole (A52) is 1 to 50 molar equivalents, preferably 1 to 20 molar equivalents, more preferably 5 to 10 molar equivalents to the compound (D4).

The condensing agent is not particularly limited, but is preferably N,N'-dicyclohexylcarbodiimide (DCC), 1-ethyl-(3-dimethylaminopropyl)carbodiimide hydrochloride (EDC·HCl), 2-(7-azobenzotriazole)-N,N,N',N'-tetramethyluronium hexafluorophosphate (HATU) or benzotriazolyl-N,N,N',N'-tetramethyluronium hexafluorophosphate (HBTU). Most preferable is DCC. The amount of the condensing agent is commonly 1 to 20 molar equivalents, preferably 5 to 10 molar equivalents to the compound (D4). A suitable amount of catalyst such as 4-dimethylaminopyridine can be added into the reaction.

The solvent can be no solvent or an aprotic solvent. The aprotic solvent can be toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide. More preferable is tetrahydrofuran, dichloromethane, dimethyl sulfoxide or dimethylformamide.

As used herein, the base is usually an organic base such as triethylamine, pyridine, 4-dimethylaminopyridine, imidazole or diisopropylethyl amine, and more preferable is triethylamine or pyridine. The amount of the base is 1 to 50 molar equivalents, preferably 1 to 10 molar equivalents, more preferably 3 to 5 molar equivalents relative to N-hydroxy succinimide (A12), phenol (A22, A32) or N-hydroxy-triazole (A52).

The reaction temperature is 0 to 200 °C, preferably 0 to 100 °C, more preferably 25 to 80 °C. The reaction time is preferably 10 mins to 48 hours, more preferably 30 mins to 24 hours. The resulting product can be purified by a purification means such as extraction, recrystallization, adsorption treatment, precipitation, reverse precipitation, membrane dialysis or supercritical extraction.

### 2.2 Preparation of monofunctional branched PEG with R selected from group B

The sulfonate derivative (B1, wherein q is 0) can be obtained via the esterification reaction between the intermediate compound (3) and a sulfonyl chloride (B11) under a basic condition.

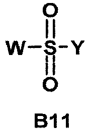

Herein, W represents Cl, Br or I, and is preferably Cl. Y is a hydrocarbon group having 1 to 10 carbon atoms which may contain fluorine. Y is preferably a methyl group, an ethyl group, a propyl group, an isopropyl group, a butyl group, a tertiary butyl group, a pentyl group, a hexyl group, a heptyl group, an octyl group, a nonyl group, a decyl group, a vinyl group, a phenyl group, a benzyl group, a p-methylphenyl group, a trifluoromethyl group, a 2,2,2-trifluoroethyl group or a 4-(trifluoromethoxy)phenyl group, more preferably a methyl group, a p-methylphenyl group, a 2,2,2-trifluoroethyl group, a trifluoromethyl group or a vinyl group.

The amount of sulfonyl chloride (B11) is 1 to 50 molar equivalents, preferably 1 to 20 molar equivalents relative to the intermediate compound (3). The solvent can be no solvent or an aprotic solvent. The aprotic solvent can be toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide, preferably tetrahydrofuran, dichloromethane, dimethyl sulfoxide or dimethylformamide.

As used herein, the base to be used can be an organic base (e.g., triethylamine, pyridine, 4-dimethylaminopyridine, imidazole or diisopropylethyl amine) or an inorganic base (e.g., sodium carbonate, sodium hydroxide, sodium bicarbonate, sodium acetate, potassium carbonate or potassium hydroxide). The base is preferably an organic base, more preferably triethylamine or pyridine. The amount of the base is 1 to 50 molar equivalents, preferably 1 to 10 molar equivalents, more preferably 10 to 15 molar equivalents relative to sulfonyl chloride (B11),

The reaction temperature is 0 to 200 °C, preferably 0 to 100 °C, more preferably 25 to 80 °C. The reaction time is preferably 10 mins to 48 hours, more preferably 30 mins to 24 hours. The resulting product can be purified by a purification means such as extraction, recrystallization, absorption treatment, precipitation, reverse precipitation, membrane dialysis or supercritical extraction.

In the case that R is a derivative of group B, q is preferably 0. When q equals unity, the preparation method similar to that when q is 0 is preferred. The relevant methods are well known by those skilled in the art, and no more will be repeated here.

### 2.3 Preparation of monofunctional branched PEG with R selected from group C

### a: Preparation of the thiol derivative (C2)

The thiol derivative (C2) can be obtained by reacting the intermediate compound (3) with a thiourea compound. 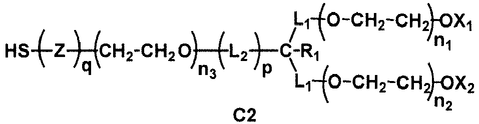 Wherein X₁, X₂, R₁, n₁, n₂, n₃, Z, L₁, L₂, p and q are the same as the above-mentioned.

The reaction can be carried out in a solvent or without any solvent. The solvent is not particularly limited but is preferably water, toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide, further preferably water, tetrahydrofuran, dichloromethane or acetonitrile. The amount of the thiourea compound is 1 to 50 molar equivalents, preferably 1 to 10 molar equivalents, more preferably 5 to 8 molar equivalents relative to the intermediate compound (3). The reaction temperature is preferably 0 to 150 °C, more preferably 20 to 100 °C, further preferably 25 to 80 °C. The reaction time is preferably 10 mins to 48 hours, more preferably 30 mins to 24 hours. The thiol derivative (C2) can be obtained via basic hydrolysis following the above reaction. The product formed can be purified by a purification means such as extraction, recrystallization, adsorption treatment, precipitation, reverse precipitation, membrane dialysis or supercritical extraction.

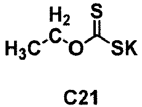

Additionally, the thiol derivative (C2) can also be obtained by reaction between the intermediate compound (3) and the compound (C21) followed by a decomposition with primary amine. The reaction can be carried out in a solvent or without any solvent. The solvent is not particularly limited, but is preferably an aprotic solvent. The aprotic solvent can be toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide, preferably tetrahydrofuran, dichloromethane, dimethyl sulfoxide or dimethyl formamide.

The amount of the compound (C21) is 1 to 50 molar equivalents, preferably 1 to 20 molar equivalents, more preferably 5 to 10 molar equivalents relative to the intermediate compound (3). The reaction temperature is preferably 0 to 150 °C, more preferably 25 to 80 °C. The reaction time is preferably 10 mins to 48 hours, more preferably 30 mins to 24 hours. And then basic hydrolysis with a primary amine is carried out in an aprotic solvent. The primary amine is preferably ammonia, methylamine, ethylamine, propylamine, butylamine, pentylamine, hexylamine, cyclohexylamine, ethanolamine, propanolamine or butanolamine. Because the mercapto group tends to be oxidized, the reaction should proceed in the absence of oxygen. The product formed can be purified by a purification means such as extraction, recrystallization, adsorption treatment, precipitation, reverse precipitation, membrane dialysis or supercritical extraction.

### b: Synthesis of the amine derivative

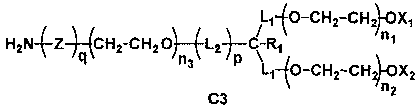

Wherein X₁, X₂, R₁, n₁, n₂, n₃, Z, L₁, L₂, p and q are the same as the above-mentioned.

The amine derivative (C3) can be synthesized in the following manner: the intermediate compound (3) is coupled with acrylonitrile or the like via base catalysis in advance, and then the cyano group of the resulting compound is reduced to the corresponding amine compound using palladium or nickel as a catalyst in a high-pressure reactor. The reaction can be carried out in a solvent or without any solvent. The solvent is not particularly limited, but is preferably water, 1,4-dioxane or combination thereof. The Base can be an organic base (e.g., triethylamine, pyridine, 4-dimethylaminopyridine, imidazole or diisopropylethyl amine) or an inorganic base (e.g., sodium carbonate, sodium hydroxide, sodium bicarbonate, sodium acetate, potassium carbonate or potassium hydroxide). The base is preferably an inorganic base, more preferably sodium hydroxide or potassium hydroxide. The amount of the base is not particularly limited, but is preferably 5 to 10 molar equivalents to the intermediate compound (3). The amount of acrylonitrile or the like is preferably 1 to 20 molar equivalents, more preferably 5 to 15 molar equivalents relative to the intermediate compound (3), and the amount increases with the molecular weight of the intermediate compound (3). Furthermore, in the case that acrylonitrile is used as solvent, the reaction temperature is -50 to 100 °C, preferably 20 to 60 °C, and the reaction time is preferably 10 mins to 48 hours, more preferably 30 mins to 24 hours.

With regard to the step of hydrogenative reduction reaction, the solvent is not particularly limited, but is preferably toluene, methanol or ethanol. The ratio of the palladium or nickel catalyst is not particularly limited, but is preferably 0.05 to 30 % by weight, more preferably 0.5 to 20% by weight to the nitrile compound. The reaction temperature is 20 to 200 °C, preferably 50 to 150 °C. The hydrogen pressure is preferably 2 to 10 MPa, more preferably 3 to 8 MPa. The reaction time is preferably 10 mins to 48 hours, more preferably 30 mins to 24 hours. Moreover, in order to inhibit dimerization, ammonia may be added to the reaction system. In the case of adding ammonia, ammonia pressure is preferably 0.1 to 3 MPa, more preferably 0.3 to 2 MPa. The product formed can be purified by a purification means such as extraction, recrystallization, adsorption treatment, precipitation, reverse precipitation, membrane dialysis or supercritical extraction.

The amine derivative (C3, q is 0) can be obtained by reacting the compound (B1) with ammonia water in aqueous ammonia solution at a concentration of 1 to 40% by weight, preferably 10 to 40% by weight. The amount of ammounia water is 1 to 300 parts by weight, preferably 100 to 200 parts by weight of the compound (B). The reaction temperature is 25 to 300, preferably 60 to 100 °C. The reaction time is preferably 10 mins to 48 hours, more preferably 30 mins to 24 hours. The resulting product can be purified by a purification means such as extraction, recrystallization, adsorption treatment, precipitation, reverse precipitation, membrane dialysis or supercritical extraction. 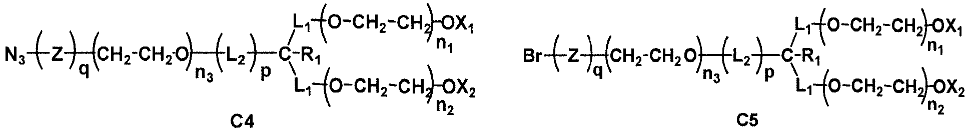 Wherein X₁, X₂, R₁, n₁, n₂, n₃, Z, L₁, L₂, p and q are the same as the above-mentioned.

Besides, the compound (C4) or (C5) can also be synthesized by reacting the compound (B1) with an azide salt or a bromide salt. The azide salt is not particularly limited as long as free azide ions exist in solvent. The azide salt is preferably sodium azide or potassium azide. Similarly, the bromide salt is not particularly limited as long as free bromide ions exist in solvent. The bromide salt is preferably sodium bromide or potassium bromide. The reaction solvent is not particularly limited, but is preferably water, ethanol, acetonitrile, dimethyl sulfoxide, dimethylformamide or dimethylacetamide, more preferably water or dimethyl formamide. The amount of the azide salt or the bromide salt is 1 to 50 molar equivalents, preferably 5 to 20 molar equivalents, more preferably 10 to 15 molar equivalents to the compound (B1). The reaction temperature is preferably 10 to 300 °C, more preferably 100 to 150 °C. The reaction time is preferably 10 mins to 48 hours, more preferably 30 mins to 24 hours. The product formed can be purified by a purification means such as extraction, recrystallization, adsorption treatment, precipitation, reverse precipitation, membrane dialysis or supercritical extraction.

### 2.4 Preparation of monofunctional branched PEG with R selected from group D

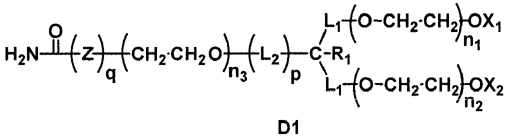

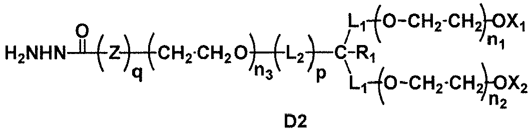

Wherein X₁, X₂, R₁, n₁, n₂, n₃, Z, L₁, L₂, p and q are the same as the above-mentioned.

The polyethylene glycol derivative (D1), (D2) or (D4) can be prepared through the following method: the intermediate compound (3) is deprotonated in advance, then undergoes substitution with an α-haloacetate ester compound, and finally is subjected to hydrolysis or aminolysis with the corresponding nucleophilic reagent.

Step A: deprotonation of intermediate compound (3). The base for deprotonating intermediate compound (3) is not particularly limited, but is preferably sodium metal, potassium metal, sodium hydride, potassium hydride, sodium methoxide, potassium methoxide, potassium tert-butoxide or diphenylmethyl potassium, more preferably sodium hydride or diphenylmethyl potassium. The amount of the base is 5 to 20 molar equivalents, preferably 8 to 15 molar equivalents to the intermediate compound (3). When the amount of the base is less than 5 molar equivalents to the intermediate compound (3), the deprotonation and thus the substitution may not sufficiently proceed. The deprotonation temperature is preferably 10 to 50 °C. When the temperature is lower than 10 °C, the deprotonation may not sufficiently proceed to result in a low ratio of functionalization.

The deprotonation time is preferably 10 minutes to 24 hours, and varies with the base to be used. A weak base or a base with relatively low solubility in an organic solvent (e.g., sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour.

Step B: addition of an α-haloacetate ester compound followed by a substitution reaction to obtain intermediate (10). 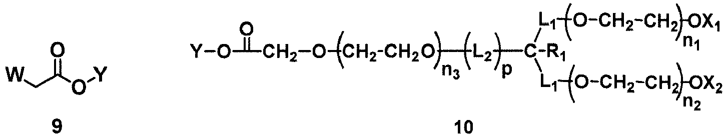 Wherein X₁, X₂, R₁, n₁, n₂, n₃, Z, L₁, L₂ and p are the same as the above-mentioned.

W is Cl, Br or I, preferably Br or I. Y is a hydrocarbon group having 1 to 10 carbon atoms which may contain fluorine. Y is preferably a methyl group, an ethyl group, a propyl group, an isopropyl group, a butyl group, a tertiary butyl group, a pentyl group, a hexyl group, a heptyl group, an octyl group, a nonyl group, a decyl group, a vinyl group, a phenyl group, a benzyl group, a p-methylphenyl group, a trifluoromethyl group, a 2,2,2-trifluoroethyl group or a 4-(trifluoromethoxy)phenyl group, more preferably a methyl group, a p-methylphenyl group, a 2,2,2-trifluoroethyl group, a trifluoromethyl group or a vinyl group.

The amide bond of (D1), the hydrazide bond of (D2) and the carboxyl group of (D4) can be formed by reacting the compound (10) with ammonia, hydrazine hydrate and basic solution, respectively.

In the case of forming the amide bond of (D1), the concentration of ammonia water is 1 to 40% by weight, preferably 25 to 35% by weight. The amount of ammonia water is 1 to 300 parts by weight, preferably 100 to 200 parts by weight of the compound (B1). The reaction temperature is 25 to 100 °C, preferably 25 to 60 °C. The reaction time is preferably 10 mins to 48 hours, more preferably 30 mins to 24 hours.

In the case of forming the hydrazide bond of (D2), the concentration of hydrazine hydrate is 1 to 80% by weight, preferably 50% to 80% by weight. The amount of hydrazine hydrate water is 1 to 300 parts by weight, preferably 50 to 100 parts by weight of the compound (B1). The reaction temperature is 25 to 100 °C, preferably 25 to 60 °C. The reaction time is preferably 10 mins to 48 hours, more preferably 30 mins to 24 hours.

In the case of forming the carboxyl group of (D4), the base is an inorganic base such as sodium hydroxide, potassium hydroxide or barium hydroxide. The concentration of the base is 0.1 to 10 mol/L, preferably 1 to 5 mol/L. The reaction temperature is 0 to 100 °C, preferably 40 to 80 °C. The reaction time is preferably 10 mins to 48 hours, more preferably 30 mins to 24 hours.

The product formed can be purified by a purification means such as extraction, recrystallization, adsorption treatment, precipitation, reverse precipitation, membrane dialysis or supercritical extraction.

### Preparation of monofunctional branched PEG with R selected from group E

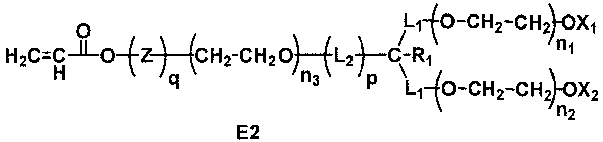

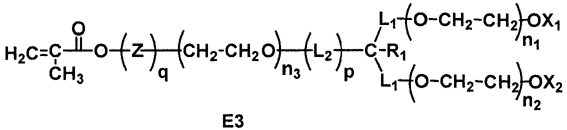

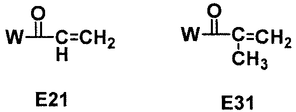

Wherein X₁, X₂, R₁, n₁, n₂, n₃, Z, L₁, L₂, p and q are the same as the above-mentioned, and W is Cl, Br or I, preferably Cl or Br.

The compound (E2) or (E3) can be obtained by reacting the deprotonated form of the PEG intermediate (3) with a corresponding halide group (E21) or (E31). With regard to the deprotonation of the PEG intermediate (3), the base to be used is not particularly limited, but is preferably sodium metal, potassium metal, sodium hydride, sodium methoxide, potassium tert-butoxide or diphenylmethyl potassium, more preferably sodium hydride or diphenylmethyl potassium. The amount of the base is 5 to 20 molar equivalents, preferably 8 to 15 molar equivalents to the intermediate compound (3). When the amount of the base is less than 5 molar equivalents to the intermediate compound (3), the deprotonation and thus the substitution may not sufficiently proceed. The deprotonation temperature is preferably 10 to 50 °C. When the temperature is lower than 10 °C, the deprotonation may not sufficiently proceed to result in a low ratio of functionalization.

The reaction solvent is not particularly limited, but is preferably aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

The deprotonation time is preferably 10 minutes to 24 hours, varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour.

The amount of the halide compound (E21) or (E31) is 1 to 50 molar equivalents, preferably 5 to 10 molar equivalents to the intermediate compound (3). The reaction temperature is 25 to 100 °C, preferably 25 to 60 °C. The reaction time is preferably 10 mins to 48 hours, more preferably 30 mins to 24 hours.

The product formed can be purified by a purification means such as extraction, recrystallization, adsorption treatment, precipitation, reverse precipitation, membrane dialysis or supercritical extraction.

### 2.5 Preparation of monofunctional branched PEG with R selected from group F

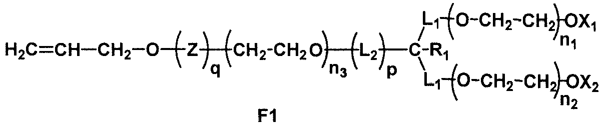

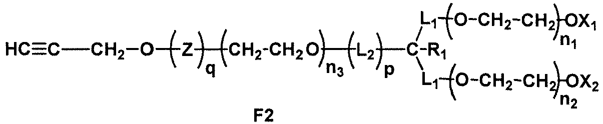

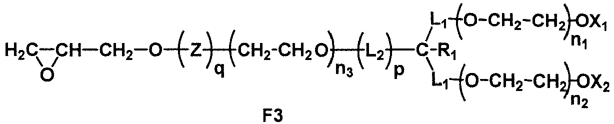

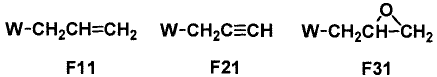

Wherein X₁, X₂, R₁, n₁, n₂, n₃, Z, L₁, L₂, p and q are the same as the above-mentioned, and W is Cl, Br or I, preferably Cl or Br.

The compound (F1), (F2) or (F3) can be obtained by reacting the deprotonated form of the PEG intermediate (3) with the corresponding halide group (F11), (F21) or (F31). The base used for deprotonating the intermediate compound (3) is not particularly limited, but is preferably sodium metal, potassium metal, sodium hydride, potassium hydride, sodium methoxide, potassium tert-butoxide or diphenylmethyl potassium, more preferably sodium hydride or diphenylmethyl potassium. The amount of the base is 5 to 20 molar equivalents, preferably 8 to 15 molar equivalents to the intermediate compound (3). When the amount of the base is less than 5 molar equivalents to the intermediate compound (3), the deprotonation and thus the substitution may not sufficiently proceed. The deprotonation temperature is preferably 10 to 50 °C. When the temperature is lower than 10 °C, the deprotonation may not sufficiently proceed to result in a low ratio of functionalization.

The reaction solvent is not particularly limited, but is preferably aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

The deprotonation time is preferably 10 minutes to 24 hours, varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour.

The amount of the halide compound (F11), (F21) or (F31) is 1 to 50 molar equivalents, preferably 5 to 10 molar equivalents to the intermediate compound (3). The reaction temperature is 25 to 100 °C, preferably 25 to 60 °C. The reaction time is preferably 10 mins to 48 hours, more preferably 30 mins to 24 hours.

The product formed can be purified by a purification means such as extraction, recrystallization, adsorption treatment, precipitation, reverse precipitation, membrane dialysis or supercritical extraction.

### 2.6 Preparation of monofunctional branched PEG with R selected from group G

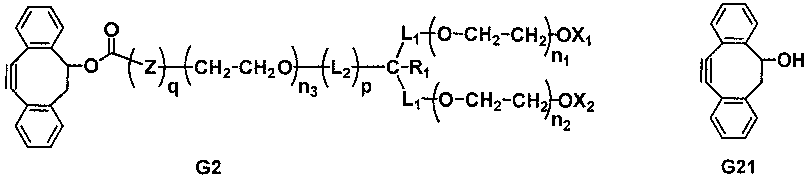

Wherein X₁, X₂, R₁, n₁, n₂, n₃, Z, L₁, L₂, p and q are the same as the above-mentioned.

Taking G2 for example, such a compound can be prepared by the condensation reaction between the polyethylene glycol acid derivative (D4) and an alcohol (G21). The amount of the alcohol (G21) is 1 to 50 molar equivalents, preferably 1 to 20 molar equivalents, more preferably 5 to 10 molar equivalents to the compound (D4),

The condensing agent is not particularly limited but is preferably DCC, EDC, HATU or HBTU, more preferably DCC or HATU. In general, the amount of the condensing agent is 1 to 20 molar equivalents, preferably 5 to 10 molar equivalents to the substrate. A suitable amount of catalyst such as 4-dimethylaminopyridine can be added to the reaction system. The solvent can be no solvent or an aprotic solvent. As used herein, the aprotic solvent can be toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide, preferably tetrahydrofuran, dichloromethane, dimethyl sulfoxide or dimethyl formamide.

Usually, the base is an organic base such as triethylamine, pyridine, 4-dimethylaminopyridine, imidazole or diisopropylethyl amine, preferably triethylamine or pyridine. The amount of the base is 1 to 50 molar equivalents, preferably 1 to 10 molar equivalents, more preferably 2 to 3 molar equivalents to condensing agent.

The reaction temperature is 0 to 200 °C, preferably 0 to 100 °C, more preferably 25 to 80 °C. The reaction time is preferably 10 mins to 48 hours, more preferably 30 mins to 24 hours.

The product formed can be purified by a purification means such as extraction, recrystallization, adsorption treatment, precipitation, reverse precipitation, membrane dialysis or supercritical extraction.

### 2.7 Preparation of monofunctional branched PEG with R as aldehyde functional groups

### 2.7.1 Preparation of acetaldehyde derivatives

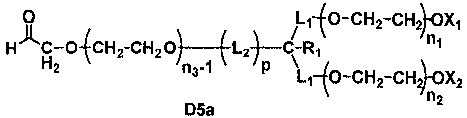

Wherein X₁, X₂, R₁, n₁, n₂, n₃, Z, L₁, L₂ and p are the same as the above-mentioned.

The polyethylene glycol aldehyde can be synthesized directly by oxidation of the intermediate compound (3). The oxidizing agent is not particularly limited, but is preferably PDC, PCC, "DCC + DMSO" or MnO₂, more preferably "DCC + DMSO". The amount of DCC is 1- to 50-fold by mole, preferably 5- to 25-fold by mole, more preferably 10- to 20-fold by mole of the intermediate compound (3). The solvent is not particularly limited, but is preferably an aprotic solvent such as toluene, benzene, xylene, acetonitrile, ethyl acetate, tetrahydrofuran, chloroform, dichloromethane, dimethyl sulfoxide, dimethylformamide or dimethylacetamide, more preferably dichloromethane or dimethyl sulfoxide. The reaction temperature is preferably -78 to 100 °C, more preferably 0 to 50 °C, further preferably 25 to 30 °C. The reaction time is preferably 10 mins to 48 hours, more preferably 30 mins to 24 hours. Additionally, the salt of a weak acid which should be added to the reaction is not particularly limited but is preferably pyridinium trifluoroacetate, triethylamine trifluoroacetate, pyridine hydrochloride, triethylamine hydrochloride, pyridine sulfate, triethylammonium sulfate or the like, more preferably pyridinium trifluoroacetate.

### 2.7.2 Preparation of derivatives of propionaldehyde and other aldehydes

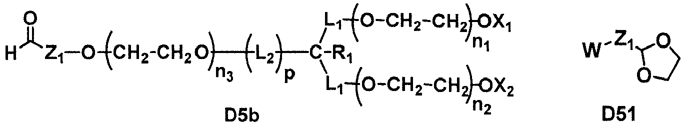

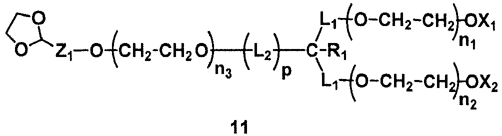

Wherein X₁, X₂, R₁, n₁, n₂, n₃, Z, L₁, L₂ and p are the same as the above-mentioned, and W is Cl, Br or I, preferably Br or I.

The derivative of propionaldehyde or an else aldehyde can be synthesized by reacting the deprotonated form of the PEG intermediate (3) with the corresponding halide compound (D51) to obtain the acetal intermediate (11) followed by hydrolysis under an acidic condition.

As used herein, the base for deprotonating the intermediate compound (3) is not particularly limited, but is preferably sodium metal, potassium metal, sodium hydride, potassium hydride, sodium methoxide, potassium tert-butoxide or diphenylmethyl potassium, more preferably sodium hydride or diphenylmethyl potassium. The amount of the base is 5 to 20 molar equivalents, preferably 8 to 15 molar equivalents to the intermediate compound (3). When the amount of the base is less than 5 molar equivalents to the intermediate compound (3), the deprotonation and thus the substitution may not sufficiently proceed. The deprotonation temperature is preferably 10 to 50 °C. When the temperature is lower than 10 °C, the deprotonation may not sufficiently proceed to result in a low ratio of functionalization.

The reaction solvent is not particularly limited, but is preferably aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

The deprotonation time is preferably 10 minutes to 24 hours, varies with the base to be used. A weak base or a base with relatively low solubility in an organic solvent (e.g. sodium methoxide, potassium methoxide, sodium hydride, potassium hydride or the like) usually calls for a long deprotonation time of 1 to 24 hours. A strong base with good solubility in an organic solvent (e.g. diphenylmethyl potassium, n-butyllithium, tert-butyllithium or the like) can be mutually fully miscible with small molecule initiators even under solvent-free conditions, and has a fast deprotonation rate. The deprotonation time of such a strong base is usually 10 minutes to 24 hours, preferably 20 minutes to 1 hour.

The amount of the halide compound (D51) is 1 to 50 molar equivalents, preferably 5 to 10 molar equivalents to the intermediate compound (3). The reaction temperature is 25 to 100 °C, preferably 25 to 60 °C. The reaction time is preferably 10 mins to 48 hours, more preferably 30 mins to 24 hours.

The deprotection of the acetal structure is carried out under an acidic condition, and the pH of the solution is preferably 1 to 4. When the pH is higher than 4, the acidity is too weak to for the protective group to be completely removed. When the pH is lower than 0, the acidity is too strong, and there is a tendency for the polyethylene glycol chain to undergo a decomposition reaction. The acid is not particularly limited, but is preferably acetic acid, phosphoric acid, sulfuric acid, hydrochloric acid or nitric acid, more preferably hydrochloric acid. The reaction solvent is not particularly limited as far as it can dissolve the reagents and the product. The solvent is preferably water. The reaction temperature is preferably 0 to 30 °C. When the temperature is lower than 0 °C, the reaction rate is relatively slow, and the protective group cannot be completely removed. When the temperature is higher than 30 °C, the decomposition reaction of the polyethylene glycol chain tends to occur.

The product formed can be purified by a purification means such as extraction, recrystallization, adsorption treatment, precipitation, reverse precipitation, membrane dialysis or supercritical extraction.

### 2.8 Preparation of monofunctional branched PEG in the case of R is maleimide.

The maleimide derivative (E1) can be prepared through either of the following method (1) or (2).
(1) An acid intermediate is obtained in advance through the ring-opening reaction between the maleic anhydride and the resulting amine derivative produced with the method shown in 2.3, and then undergoes a ring-closure reaction using acetic anhydride or sodium acetate as a catalyst to obtain the maleimide compound (E1). 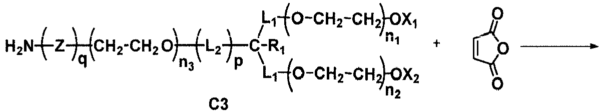 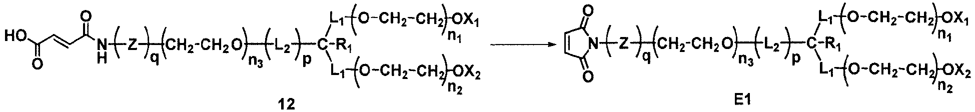 Wherein X₁, X₂, R₁, n₁, n₂, n₃, Z, L₁, L₂, p and q are the same as the above-mentioned.

The reaction solvent is not particularly limited, but it is preferably an aprotic solvent such as toluene, benzene, dimethylbenzene, acetonitrile, acetic ether, tetrahydrofuran, chloroform, dichloromethane, dimethyl sulfoxide, dimethylformamide or dimethylacetamide, more preferably toluene or tetrahydrofuran.

The amount of the maleic anhydride is preferably 1- to 100-fold by mole, more preferably 5- to 10-fold by mole of the amine compound (C3). The reaction temperature is preferably 0 to 200 °C, more preferably 25 to 150 °C. The reaction time is preferably 10 mins to 48 hours, more preferably 30 mins to 24 hours. The product formed can be purified by a purification means such as extraction, recrystallization, adsorption treatment, precipitation, reverse precipitation, membrane dialysis or supercritical extraction.

The solvent in the ring-closure reaction is not particularly limited, but is preferably an aprotic solvent or acetic anhydride. The amount of sodium acetate to be used is 0.1- to 100-fold by mole, preferably 1- to 50-fold by mole of the intermediate compound (3). The reaction temperature is preferably 0 to 200 °C, more preferably 25 to 150 °C. The reaction time is preferably 10 mins to 48 hours, more preferably 30 mins to 24 hours. The product formed can be purified by a purification means such as extraction, recrystallization, adsorption treatment, precipitation, reverse precipitation, membrane dialysis or supercritical extraction.
(2) The maleimide compound (E1) can be obtained by the condensation reaction between the aforementioned amine compound (C3) and an acid compound containing maleimide group (E11). 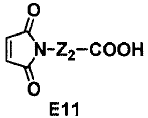 Wherein Z₂ is an alkylene or an alkylene containing at least one group which is stable under the conditions of light illumination, enzyme, acid or base, wherein each group is selected from a group consisting of an ester bond, a urethane bond, an amide bond, an ether bond, a double bond, a triple bond, a carbonate bond and a secondary amino group. Z₂ is more preferably an alkylene or an alkylene containing at least one of a group consisting of an ester bond, an amide bond and a secondary amino group. As used herein, the alkylene is preferably methylene, 1,2-ethylene, 1,3-propylene, 1,2-propylene, isopropylidene, butylene, pentylene or hexylene.

The condensing agent is not particularly limited, but is preferably DCC, EDC, HATU or HBTU, more preferably DCC. In usual, the amount of the condensing agent is equimolar to 20 molar, preferably 5 molar to 10 molar to the substrate. A suitable amount of catalyst such as 4-dimethylaminopyridine can be added to the reaction system.

The reaction solvent is not particularly limited, but is preferably aprotic solvent such as toluene, benzene, xylene, acetonitrile, ethyl acetate, diethyl ether, methyl tert-butyl ether, tetrahydrofuran, chloroform, dichloromethane, dimethyl sulfoxide, dimethylformamide or dimethylacetamide, more preferably tetrahydrofuran, dichloromethane, dimethyl sulfoxide or dimethyl formamide.

The base to be used is an organic base such as triethylamine, pyridine, 4-dimethylaminopyridine, imidazole or diisopropylethyl amine, preferably triethylamine or pyridine. The amount of the base is equimolar to 50 molar, preferably equimolar to 10 molar, more preferably 2 molar to 3 molar to the condensing agent,.

The reaction temperature is 0 to 200 °C, preferably 0 to 100 °C, more preferably 25 to 80 °C. The reaction time is preferably 10 mins to 48 hours, more preferably 30 mins to 24 hours.

The product formed can be purified by a purification means such as extraction, recrystallization, adsorption treatment, precipitation, reverse precipitation, membrane dialysis or supercritical extraction.

The above-mentioned description of the structure of the monofunctional branched polyethylene glycol only provides some common examples. As for the preparation methods thereof, only routes from compound (3) are described. It should be noted that the preparation of a monofunctional branched polyethylene glycol can also be expediently realized through compound (HI) when q equals 1, meanwhile the related steps and reagents are similar to those used in the method through compound (3) and well known by those skilled in the art.

### 2.9 Preparation of PEG-modified bio-related substance.

The "bio-related substance" according to the invention represents a physiologically active substance or a modified derivative thereof. Examples of the bio-related substance include but are not particularly limited to the following substances: polypeptide, protein, enzyme, small molecule drug, dye, liposome, nucleoside, nucleotide, oligonucleotide, polynucleotide, nucleic acid, polysaccharide, steroid, lipid, phospholipid, glycolipid, glycoprotein, virus, cell and micelle. They can be classified into the following groups:

### (1) Saccharides

Saccharides are major component constituting cells and organs. As used herein, saccharides are not particularly limited, but mainly include glycolipid, glycoprotein, glycogen and the like. Glycolipid is widely distributed in the organism, and mainly has two categories including glycosyl-acylglycerid and glycosphingolipid. Specific examples of glycolipids include ceramide, cerebroside, sphingol, ganglioside, glyceryl glycolipid and the like. Glycoprotein, a kind of polyconjugates made from chains of branched oligosaccharide and polypeptides via covalent connection, is commonly secreted into body fluid or acts as a component of membrane protein, specifically including transferrin, serum ceruloplasmin, membrane-binding protein, histocompatibility antigen, hormone, carrier, lectin, antibody and the like.

### (2) Lipids

Lipids are mainly made up of two categories including fat and lipoid. As used herein, the fatty acid is not particularly limited but preferably an aliphatic acid having 12 to 22 carbon atoms. The fatty acid can be a saturated fatty acid or that containing an unsaturated bond. The lipoid includes glycolipid, phospholipid, cholesteryl ester and the like. Wherein, the phospholipid may be derived from natural phospholipid substance such as yolk or soybean, or may be synthesized phospholipid compound. Preferable examples of phospholipid include phosphatidic acid, phosphatidylcholine, phosphatidylethanolamine, cardiolipin, phosphatidylserine, phosphatidylinositol and lyso isomer thereof. Cholesterol, steroid or the like play an essential role in modulating and maintaining normal metabolism and procreation of the body, and examples include cholesterol, bile acid, sex hormone, vitamin D and the like.

### (3) Nucleic acids

Nucleic acid, one of the most basic substances of life, is a kind of biomacromolecules made by polymerization of nucleotides. It is widely discovered in all animals, plants, cells and microorganisms. The nucleic acids in body are usually combined with proteins to form nucleoproteins. According to chemical composition, nucleic acids are classified into ribonucleic acids and deoxyribonucleic acids.

### (4) Polypeptides and proteins

Protein is considered as an essential component of life. More specific examples of proteins and polypeptides include the following. Hormones such as neuohypophysial hormone, thyroid hormone, male sex hormone, female sex hormone and adrenal cortex hormone. Serum proteins such as hemoglobin and blood factors. Immunoglobulins such as IgG, IgE, IgM, IgA and IgD. Cytokines and fragments thereof such as interleukin, interferon, granulocyte-colony stimulating factor, macrophage-colony stimulating factor, granulocyte-macrophage colony stimulating factor, platelet-derived growth factor, phospholipase-activating protein, insulin, glucagon, lectin, ricin, tumor necrosis factor, epidermal growth factor, vascular endothelial growth factor, nerve growth factor, bone growth factor, insulin-like growth factor, heparin-binding growth factor, tumor growth factor, glial cell line-derived neurotrophic factor, macrophage differentiating factor, differentiation-inducing factor, leukemia inhibitory factor, amphiregulin, somatotropin, erythropoietin, hemopoietin, thrombopoietin and calcitonin. Enzymes such as proteolytic enzymes, oxidoreductases, transferases, hydrolases, lyases, isomerases, ligases, asparaginases, arginases, arginine deaminases, adenosine deaminases, superoxide dismutases, endotoxinases, catalases, chymotrypsin, lipases, uricases, elastases, streptokinases, urokinases, prourokinases, adenosine diphosphatases, tyrosinases, bilirubin oxidases, glucose oxidases, glucosases, glactosidases, glucocerebrosidases and glucouronidases. Monoclonal and polyclonal antibodies and fragments thereof, poly(amino acids) such as poly-L-lysine and poly-D-lysine. Vaccines such as hepatitis B vaccine, malaria vaccine, melanoma vaccine and HIV-1 vaccine. Antigens and viruses.

### (5) Others

A Vitamin is an organic compound in a limited amount required by human and animals to maintain normal physiological behaviors, and it must be obtained from food. Vitamin plays an important role in the processes of growth, metabolism and development of the body. More specific examples of vitamins include vitamin A, vitamin B, vitamin E, vitamin K and the like.

As used herein, the small molecule drugs are not particularly limited but more preferably include anticancer drugs and antifungal drugs. Preferable anticancer drugs include paclitaxel, adriamycin, doxorubicin, cis-platinum, daunomycin, mitomycin, vincristine, epirubicin, methotrexate, 5-fluorouracil, aclacinomycin, idamycin, bleomycin, pirarubicin, peplomycin, vancomycin, camptothecin and the like. Specific examples of antifungal drugs are not particularly limited but include amphotericin B, nystatin, fluorocytosine, miconazole, fluconazole, itraconazole, ketoconazole, peptide antifungal drugs and the like.

Other bio-related substances well known by those skilled in the art such as liposomes, cells, micelles and the like are also included in the present invention.

The reactive group of the bio-related substance reacts with the active group of the monofunctional branched polyethylene glycol to form a covalent residue group L₃ which links the bio-related substance and the branched PEG. Herein, the residue group L₃ is preferably a triazole bond, an isoxazole bond, an ether bond, an amide bond, an imide bond, an imino group, a secondary amino group, a tertiary amino group, a thioester bond, a disulfide bond, a urethane bond, a thiocarbonate bond a sulfonate bond, a sulfamide bond, a carbamate bond, a tyrosine group, a cysteine group, a histidine group or the combination thereof.

The structure of the residue L₃ group is depended on the reactive group of the bio-related substance and the functional group of PEG derivative. Specific examples are listed below. In the case of reacting an amino group of a bio-related substance with an active ester group, a carbonic active ester group, a sulfonate group, an aldehyde group, an□α,β-unsaturated bond or a carboxylic acid group of a functional PEG, an amide bond, an urethane bond, an amino group, an imide group which can be further reduced to a secondary amino group, an amino group or an amide bond is formed as a linking group in the resulting PEG-modified bio-related substance. In the case of reacting a mercapto group of a bio-related substance with an active ester group, a carbonic active ester group, a sulfonate group, a mercapto group, a maleimide group, an aldehyde group, an α,β-unsaturated bond or a carboxyl group of a PEG derivative, a thioester bond, a thiocarbonate bond, a thioether bond, a disulfide bond, a thioether bond, a hemithioacetal bond, a thioether bond or a thioester bond is formed as a linking group in the resulting PEG-modified bio-related substance. In the case of reacting an unsaturated bond of a bio-related substance with a mercapto group of a PEG derivative, a thioether bond is formed as a linking group in the resulting PEG-modified substance. In the case of reacting a carboxyl group of a bio-related substance with a mercapto group or an amino group of a PEG derivative, a thioester bond or an amide bond is formed as a linking group in the resulting PEG-modified substance.

In the following, the monofunctional branched polyethylene glycol of the present invention and production process thereof are described more specifically with reference to EXAMPLES. The specific examples are given to further illustrate the invention, but should not be regarded as the limitation of the invention.

### EXAMPLES

### Example 1: the preparation of monofunctional branched PEG with R selected from group H

### Preparation of compound H1-1

In this example, the compound in group H is selected as follows: L₁ = CH₂, L₂ = CH₂, R₁ = H, X₁ = CH₃, X₂ = CH₃, p = 1, q = 0, and TBS is selected as the protective group of the terminal hydroxyl group of the small molecule initiator. The designed total molecular weight is approximately 20000, wherein the molecular weight of two branch chains is approximately 2 × 8500 = 17000 corresponding to n₁ ≈ n₂ ≈ 193; and the molecular weight of the main chain is approximately 3000 corresponding to n₃ ≈ 68. 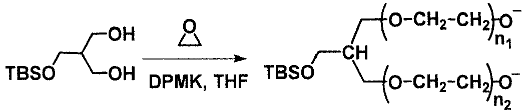
a, Into a sealed reactor in an anhydrous and oxygen-free atmosphere, tetrahydrofuran (250 mL), small molecule initiator (2.532 mmol) and diphenylmethyl potassium (4.0 mmol) were added in sequence;
b, After a calculated amount of ethylene oxide (50 mL) was added thereto, the whole was heated stepwisely to 60 °C, followed by 48 hours of reaction at 60 °C.
c, After the reaction was finished, excess diphenylmethyl potassium (40 mmol) and excess methyl iodide (100 mmol) were added in sequence, followed by 12 hours of reaction at 30°C. After the completion of the reaction, open the reactor. The product in the solvent was concentrated, and then precipitated with absolute ether at 0°C. The crystals were collected by filtration and dried to obtain the intermediate 6-1 containing a terminal hydroxyl group protected with a siloxane group on the main chain. 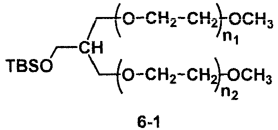
   ¹H NMR data of the intermediate 6-1 in this example are as follows:
   ¹H NMR (CDCl₃) δ (ppm): 0.21 (-Si(C***H***₃)₂), 0.98 (-SiC(C***H***₃)₃), 2.51 (-C***H***(CH₂)₃-), 3.35 (*C**H**₃*O-), 3.40-3.80 (-C***H**₂*C***H**₂*O*-,* -CH-C***H***₂-OSi-); Mₙ = 17000, PDI = 1.03.
d, Into a clean container, the intermediate 6-1 obtained in step c was added and then dissolved with tetrahydrofuran. Tetra-butyl ammonium fluoride (TBAF) was added thereto, and the reaction was conducted overnight to obtain the intermediate (7) with a terminal hydroxyl group being deprotected.
   ¹H NMR data of the intermediate 7 in this example are as follows:
   ¹H NMR (CDCl₃) δ (ppm): 2.52 (-C***H***(CH₂)₃-), 3.35 (C***H**₃*O-), 3.40-3.80 (-C***H**₂*C***H**₂*O*-,* -CK-C***H***₂O-); Mₙ = 17000, PDI = 1.03.
e, Step (a) and (b) were repeated followed by an addition of excess proton source (e.g., methanol) to obtain the following compound H1-1 (L₁ = CH₂, L₂ = CH₂, R₁ = H, X₁ = X₂ = CH₃, p =1,q=0). 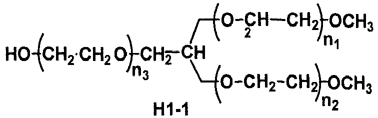
   ¹H NMR data of the compound H1-1 are as follows:
   ¹H NMR (CDCl₃) δ (ppm): 2.51 (-C***H***(CH₂)₃-), 3.35 (C***H**₃*O-), 3.40-3.80 (-C***H**₂*C***H**₂*O*-,* -CHC***H***₂O-); Mₙ = 20000, PDI = 1.05 (molecular weight is around 2 × 8500 + 3000 = 20000, wherein the molecular weight of the main chain is approximately 3000) .

### Preparation of compound Hl-2

In this example, the compound in group H is selected as follows: L₁ = CH₂, L₂ = CH₂, R₁ = H, X₁ = CH₃, X₂ = CH₃, p = 1, q = 0, and EE is selected as the protective group of the terminal hydroxyl group of the small molecule initiator. The designed total molecular weight is around 40000, wherein the molecular weight of two branch chains is approximately 2*8500 = 17000 corresponding to n₁ ≈ n₂ ≈ 193; and the molecular weight of the main chain is approximately 23000 corresponding to n₃ ≈ 522. 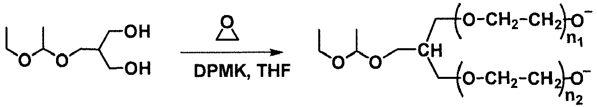
a, Into a sealed reactor in an anhydrous and oxygen-free atmosphere, tetrahydrofuran (250 mL), initiator (2.532 mmol) and diphenylmethyl potassium (4.0 mmol) were added in sequence;
b, After a calculated amount of ethylene oxide (50 mL) was added thereto, the whole was heated stepwisely to 60 °C, followed by 48 hours of reaction at 60 °C.;
c, After the reaction was finished, excess diphenylmethyl potassium (40 mmol) and excess methyl iodide (100 mmol) were added in sequence, followed by 12 hours of reaction at 30°C. After the completion of the reaction, open the reactor. The product in the solvent was concentrated, and then precipitated with absolute ether at 0°C. The crystals were collected by filtration and dried to obtain the intermediate 6-2 containing a terminal hydroxyl group protected with an acetal group on the main chain. 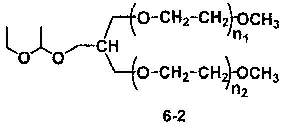
   ¹H NMR data of the intermediate 6-2 in this example are as follows:
   ¹H NMR (CDCl₃) δ (ppm): 1.22 (-OCH₂C***H***₃), 1.30 (-OCH(O)C***H***₃), 2.51 (-C***H***(CH₂)₃-), 3.35 (C***H**₃*O-), 3.40-3.80 (-C***H**₂*C***H**₂*O*-,* -CHC***H***₂O-, OC***H***₂CH₃), 4.75 (-OC***H***CH₃(OCH₂)); Mn = 17000, PDI = 1.03.
d, Into a clean container, the V-shaped PEG obtained in step c was added and then dissolved with methanol. The solution was adjusted to pH 1.0 with hydrochloric acid (1 M) followed by 4 hours of reaction to obtain the intermediate (7) with a terminal hydroxyl group being deprotected.
   ¹H NMR data of the intermediate 7 in this example are as follows:
   ¹H NMR (CDCl₃) δ (ppm): 2.52 (-C***H***(CH₂)₃-), 3.35 (C***H**₃*O-), 3.40-3.80 (-C***H**₂*C***H**₂*O*-,* -CH-C***H***₂O-); Mₙ = 17000, PDI = 1.03.
e, Step (a) and (b) were repeated followed by an addition of excess proton source such as methanol to obtain the following compound H1-2 (L₁ = CH₂, L₂ = CH₂, R₁ = H, X₁ = X₂ = CH₃, p = 1, q = 0). 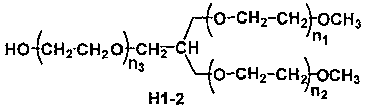
   ¹H NMR data of the compound H1-2 are as follows:
   ¹H NMR (CDCl₃) δ (ppm): 2.51 (-C***H***(CH₂)₃-), 3.35 (C***H**₃*O-), 3.40-3.80 (-C***H**₂*C***H**₂*O*-,* -CHC***H***₂O-); Mₙ = 40000, PDI = 1.05 (molecular weight is approximately 2 × 8500 + 23000 = 40000, wherein the molecular weight of the main chain is approximately 23000) .

### Preparation of compound H1-3

In this example, the compound in group H is selected as follows: L₁ = CH₂, L₂ = CH₂, R₁ = H, X₁ = CH₃, X₂ = CH₃, p = 1, q = 0, and Bn is selected as the protective group of the terminal hydroxyl group of the small molecule initiator. The designed total molecular weight is 30000, wherein the molecular weight of two branch chains is approximately 2×10000 = 20000, corresponding to n₁ ≈ n₂≈ 227; the molecular weight of the main chain is approximately 10000 corresponding to n₃≈ 227. 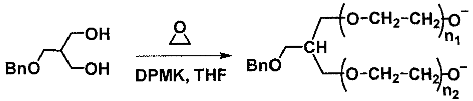
a, Into a sealed reactor in an anhydrous and oxygen-free atmosphere, tetrahydrofuran (250 mL), initiator (2.02 mmol) and diphenylmethyl potassium (3.2 mmol) were added in sequence;
b, After a calculated amount of ethylene oxide (50 mL) was added thereto, the whole was heated stepwisely to 60 °C, followed by 48 hours of reaction at 60 °C.
c, After the reaction was finished, excess diphenylmethyl potassium (32 mmol) and excess methyl iodide (54 mmol) were added in sequence, followed by 12 hours of reaction at 30°C. After the completion of the reaction, open the reactor. The product in the solvent was concentrated, and then precipitated with absolute ether at 0°C. The crystals were collected by filtration and dried to obtain the intermediate 6-3 containing a terminal hydroxyl group protected with a benzyl group on the main chain. 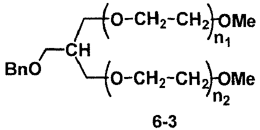
   ¹H NMR data of the intermediate 6-3 in this example are as follows:
   ¹H NMR (CDCl₃) δ (ppm): 2.51 (-C***H***(CH₂)₃), 3.35 (*C**H**₃*O-), 3.40-3.80 (-C***H**₂*C***H**₂*O*-,* -CHC***H***₂O-), 4.70 (OC***H***₂C₆H₅), 7.35-7.50 (OCH₂C₆***H***₅); Mₙ = 20000, PDI = 1.05.
d, Into a clean container fitted with a nitrogen-introducing tube, the V-shape PEG obtained in step c which has been treated by azeotropic removal of water and 5% Pd/C were added in the same amount of weight. With introducing nitrogen thereinto, cyclohexene was added followed by 4 hours of reaction at 40°C. The liquid was filtrated, washed with ethyl acetate, concentrated, and finally precipitated with diethyl ether to obtain the intermediate 7 with a terminal hydroxyl group being deprotected.
   ¹H NMR data of the intermediate 7 in this example are as follows:
   ¹H NMR (CDCl₃) δ (ppm): 2.52 (-C***H***(CH₂)₃-), 3.35 (C***H**₃*O-), 3.40-3.80 (-C***H**₂*C***H**₂*O*-,* -CH-C***H***₂O-); Mₙ = 20000, PDI = 1.05.
e, Step (a) and (b) were repeated followed by an addition of excess proton source such as methanol to obtain the following compound H1-3 (L₁ = CH₂, L₂ = CH₂, R₁ = H, X₁ = X₂ = CH₃, p = 1, q = 0). 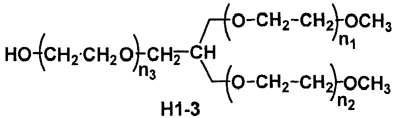
   ¹H NMR data of the compound H1-3 are as follows:
   ¹H NMR (CDCl₃) δ (ppm): 2.51 (-C***H***(CH₂)₃-), 3.35 (C***H**₃*O-), 3.40-3.80 (-C***H**₂*C***H**₂*O-, -CHC***H***₂O-); Mₙ = 30000, PDI = 1.10 (molecular weight is approximately 2 × 10000 + 10000 = 30000, wherein the molecular weight of the main chain is approximately 10000) .

### Example 2: the preparation of active ester derivatives

### Synthesis of active ester compound A1-1

In the case of synthesizing the active ester compound (A1-1), L₁ = CH₂, L₂ = CH₂, R₁ = H, X₁ = CH₃, X₂ = CH₃, Z = OCH₂CH₂O, p = 1, q = 1, and the molecular weight is approximately 20000, wherein the value of n₁, n₂, n₃ are the same as that in compound H1-1. In this example, the corresponding active ester is prepared by the reaction of the terminal hydroxyl group of compound H1-1 with the carbonate.

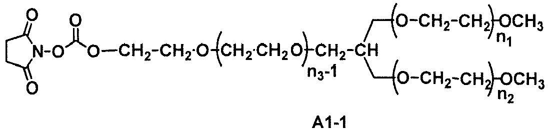

Into a dry and clean 1 L round-bottom flask, 40 g of branched PEG obtained in example 1 (H1-1 treated by azeotropic removal of water with toluene), 500 mL of acetonitrile, 40 mL of triethylamine and 10 g of N,N'-disuccinimidyl carbonate were added, followed by 24 hours of reaction. The resulting product was concentrated, and recrystallized from isopropanol to obtain the active ester compound (A1-1) in a white solid state.
¹H NMR data of the active ester compound Al-1 are as follows:
¹H NMR (CDCl₃) δ (ppm): 2.51 (-C***H***(CH₂)₃-), 2.80 (-(O=)CC***H**₂*C***H**₂*C(=O)-), 3.35 (C***H**₃*O-), 3.40-3.80 (-C***H**₂*C***H**₂*O*-,* -CHC***H***₂O-), 4.15 (-C***H**₂*OCO-).

### Synthesis of p-nitrophenyl carbonate compound A2-1

In the case of synthesizing the p-nitrophenyl carbonate compound (A2-1), L₁ = CH₂, L₂ = CH₂, R₁ = H, X₁ = CH₃, X₂ = CH₃, Z = OCH₂CH₂O, p =1, q = 1, and the molecular weight is approximately 20000, wherein the value of n₁, n₂, n₃ are the same as that in compound H1-1.

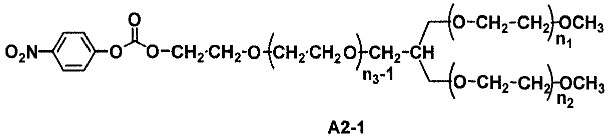

Into a 1 L round-bottom flask fitted with a condenser, 40 g of branched PEG obtained in example 1 (H1-1 treated by azeotropic removal of water with toluene), 500 mL of toluene, 40 mL of triethylamine and 10 g of 4-nitrophenyl chloroformate were added, followed by 24 hours of reaction at 80 °C. The resulting product was filtrated, concentrated, and recrystallized from isopropanol to obtain the p-nitrophenyl carbonate compound (A2-1).
¹H NMR data of the p-nitrophenyl carbonate compound A2-1 are as follows:
¹H NMR (CDCl₃) δ (ppm): 2.51 (-C***H***(CH₂)₃), 3.35 (C***H**₃*O-), 3.40-3.80 (-C***H**₂*C***H**₂*O*-,* -CHC***H***₂O-), 4.30-4.50 (-C***H**₂*OCO-), 7.40 (-C₆***H***₄NO₂), 8.28 ((-C₆***H***₄NO₂).

### Synthesis of active ester compound A1-2

In the case of synthesizing the active ester compound (A1-2), L₁ = CH₂, L₂ = CH₂, R₁ = H, X₁ = CH₃, X₂ = CH₃, Z = OCH₂, p = 1, q = 1, and the molecular weight is approximately 20000.

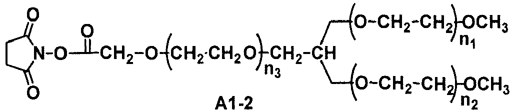

Into a dry and clean 1 L round-bottom flask fitted with a nitrogen-introducing tube, 40 g of branched PEG carboxylic acid derivative obtained in example 4 (D4-1), 20 mL of triethylamine and 10 g of N-hydroxysuccinimide were added. With introducing nitrogen thereinto, dichloromethane (200 mL) as solvent was added, and the whole was stirred until all were dissolved. 20 g of dicyclohexylcarbodiimide (DCC) in dichloromethane was added thereto, followed by 24 hours of reaction at room temperature. The resulting product was filtrated to remove undissolved substances, concentrated, and recrystallized from isopropanol to obtain the active ester compound (A1-2) in a white solid state.
¹H NMR data of the active ester compound A1-2 are as follows:
¹H NMR (CDCl₃) δ (ppm): 2.51 (-C***H***(CH₂)₃-), 2.81 (-(O=)CC***H**₂*C***H**₂*C(=O)-), 2.83 (-(O=)CC***H**₂*C***H**₂*C(=O)-), 3.35 (C***H**₃*O-), 3.40-3.80 (-C***H**₂*C***H**₂*O*-,* -CHC***H***₂O-),4.61 (-OC***H***₂COO-).

### Example 3: the preparation of sulfonate derivatives

### Synthesis of sulfonate B1-1

In the case of synthesizing the sulfonate compound (B1-1), R = OTs, L₁ = CH₂, L₂ = CH₂, R₁ = H, X₁ = CH₃, X₂ = CH₃, Z = OCH₂CH₂O, p = 1, q = 0, and the molecular weight is approximately 20000, wherein the value of n₁, n₂, n₃ are the same as that in compound H1-1.

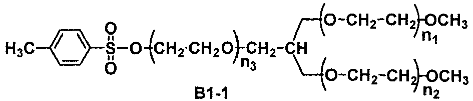

Into a dry and clean 1 L round-bottom flask fitted with a nitrogen-introducing tube, 40 g of branched PEG obtained in example 1 (H1-1) was added. With introducing nitrogen thereinto, 500 mL of anhydrous and oxygen-free dichloromethane, 20 mL of pyridine and 15 g of p-toluenesulfonyl chloride were added, followed by 24 hours of reaction at room temperature. After completion of the reaction, the solution was neutralized to pH 7 with 1 mol/L hydrochloric acid. The aqueous phase was washed with dichloromethane (50 mL thrice). The organic phase was collected, washed with saturated salt solution, dried over anhydrous sodium sulfate, filtrated, concentrated, and recrystallized to obtain the sulfonate compound (B1-1).
¹H NMR data of the sulfonate compound (Bl-1) are as follows:
¹H NMR (CDCl₃) δ (ppm): 2.42 (C***H***₃C₆H₄SO₂-), 2.51 (-C***H***(CH₂)₃), 3.35 (C***H**₃*O-), 3.40-3.80 (-C***H**₂*C***H**₂*O-, -CHC***H***₂O-), 7.30 (CH₃C₆***H**₄*SO₂-), 7.80 (CH₃C₆***H***₄SO₂-).

### Example 4

### Synthesis of mercapto derivative C2-2

In the case of synthesizing the mercapto derivative (C2-2), R = SH, L₁ = CH₂, L₂ = CH₂, R₁ = H, X₁ = CH₃, X₂ = CH₃, p = 1, q = 0, and the molecular weight is approximately 20000, wherein the value of n₁, n₂, n₃ are the same as that in compound B1-1. 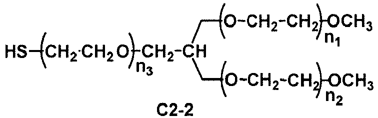
A: Into a dry and clean 1 L round-bottom flask fitted with a nitrogen-introducing tube, 40 g of branched PEG sulfonic acid ester obtained in example 3 (B1-1) was added. With introducing nitrogen thereinto, 400 mL of tetrahydrofuran and 16 mL of DMF were added, followed by stirring until the whole was dissolved. Thereafter, 10 g of ethanesulfonic acid potassium was added followed by 24 hours of reaction at room temperature. The resulting product was concentrated. Subsequently, 400 mL of dichloromethane was added thereto, and the undissolved substances were removed by filtration. The filtrate was further washed with saturated salt solution (100 mL thrice), dried, concentrated again and recrystallized from isopropanol to obtain the intermediate (C2-1) in a white or light yellow solid state. 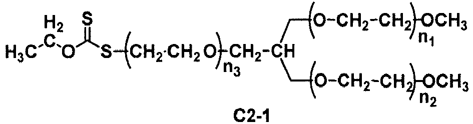
   ¹H NMR data of the intermediate C2-1 are as follows:
   ¹H NMR (CDCl₃) δ (ppm): 0.9 (C***H**₃*CH₂OCS-), 2.51 (-C***H***(CH₂)₃-), 2.82 (-OCH*₂*C***H**₂*S-), 3.35 (C***H**₃*O-), 3.40-3.80 (-C***H**₂*C***H**₂*O*-,* -CHC***H***₂O-, -SCH₂C***H***₂O-, CH₃C***H***₂OCS-).
B: Into a dry and clean 1 L round-bottom flask fitted with a nitrogen-introducing tube, 20 g of branched PEG sulfate obtained in step A (C2-1) was added. With introducing nitrogen thereinto, 200 mL of tetrahydrofuran was added, followed by stirring until the whole was dissolved. Thereafter, 10 g of n-propylamine was added followed by 24 hours of reaction at room temperature. The resulting product was concentrated, and recrystallized from oxygen-free isopropanol to obtain the mercapto derivative (C2-2) in a white or light yellow solid state.
   ¹H NMR data of thiol derivatives C2-2 are as follows:
   ¹H NMR (CDCl₃) δ (ppm): 2.51 (-C***H***(CH₂)₃-), 2.85 (-OCH*₂*C***H**₂*SH), 3.35 (C***H**₃*O-), 3.40-3.80 (-OC***H***₂*C**H**₂*O*-,* -CHC***H***₂O-, -OC***H***₂C***H***₂SH).

### Synthesis of amine derivative C3-1

In the case of synthesizing the amine derivative (C3-1), R = NH₂, L₁ = CH₂, L₂ = CH₂, R₁ = H, X₁ = CH₃, X₂ = CH₃, p = 1, q = 0, and the molecular weight is approximately 20000, wherein the value of n₁, n₂, n₃ are the same as that in compound B1-1.

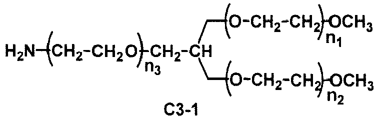

Into a dry and clean 1 L round-bottom flask, 40 g of branched PEG sulfate obtained in example 3 (B1-1) was added, followed by an addition of 800 mL of 40 wt% ammonia water. The whole was stirred until being dissolved. Thereafter, the reaction was conducted at room temperature for one week. The liquid was extracted with dichloromethane (200 mL thrice). The organic phase was collected, washed with saturated salt solution, dried, concentrated, and recrystallized to obtain the white amine derivative (C3-1).
¹H NMR data of amine derivative C3-1 are as follows:
¹H NMR (CDCl₃) δ (ppm): 2.51 (-CH(CH₂)₃-), 2.85 (-OCH₂CH₂SH), 3.35 (C*H₃*O-), 3.40-3.80 (-OC*H₂*C*H₂*O-, -CHC*H*₂O-, -OC*H*₂CH₂SH).

### Synthesis of amine derivative C3-2

In the case of synthesizing the amine derivative (C3-2), R = OCH₂CH₂CH₂NH₂, L₁ = CH₂, L₂ = CH₂, R₁ = H, X₁ = CH₃, X₂ = CH₃, Z = OCH₂CH₂CH₂, p = 1, q = 1, and the molecular weight is approximately 40000, wherein the value of n₁, n₂, n₃ are the same as that in compound H1-2. 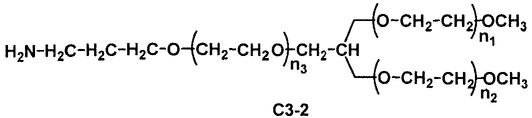
A: Into a dry and clean 1 L round-bottom flask fitted with a nitrogen-introducing tube, 40 g of branched PEG obtained in example 1 (H1-2) was added. With introducing nitrogen thereinto, 500 mL of 1,4-dixane was added. The whole was stirred until being dissolved. Thereafter, in an ice-bath, 10 g of 50% potassium hydroxide was added, and propenyl cyanide was dropwisely added, followed by 24 hours reaction at room temperature. After completion of the reaction, the solution was neutralized to pH 7 with 1 mol/L hydrochloric acid. The liquid was concentrated to remove 1,4-dixane, and added with 100 mL of deionized water. The aqueous phase was washed with dichloromethane (50 mL thrice). The organic phase was collected, washed with saturated salt solution, dried over anhydrous sodium sulfate, filtrated, concentrated, and precipitated to obtain the intermediate (F1-1). 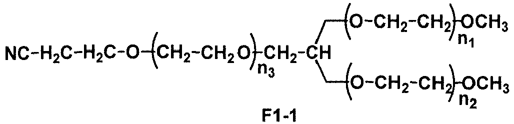
   ¹H NMR data of the intermediate F1-1 are as follows:
   ¹H NMR (CDCl₃) δ (ppm): 2.51 (-C***H***(CH₂)₃-), 2.60(-CH₂C***H***₂CN), 3.35 (C***H**₃*O-), 3.40-3.80 (-C***H***₂C***H***₂O-, -CHC***H***₂O-, -OC***H***₂CH₂CN); Mₙ = 40000, PDI = 1.10.
B: Into a high-pressure reactor, 50 g intermediate obtained in step A (F1-1) and 500 mL of toluene were added in sequence. The whole was heated until being dissolved. After dissolution, 5.0 g nickel or Pt/C was added thereto. Thereafter, ammonia gas was pressured to 0.7 MPa, and then hydrogen gas was pressured to 4.5 MPa. The reaction was conducted at 130 °C overnight. After completion of the reaction, the resulting product was filtrated, concentrated, recrystallized from isopropanol to obtain the white amine derivative (C3-2).
   ¹H NMR data of white amine derivative C3-2 are as follows:
   ¹H NMR (CDCl₃) δ (ppm): 1.81 (-CH₂C***H***₂CH₂NH₂), 2.51 (-C***H***(CH₂)₃-), 2.83 (-CH₂C***H***₂NH₂), 3.35 (C***H**₃*O-), 3.40-3.80 (-C***H**₂*C***H**₂*O*-,* -CHC***H***₂O-, -OC***H***₂CH₂NH₂); Mₙ = 40000, PDI = 1.10.

### Synthesis of hydrazide derivative D2-1

In the case of synthesizing the hydrazide derivative (D3-1), R = OCH₂CONHNH₂, L₁ = CH₂, L₂ = CH₂, R₁ = H, X₁ = CH₃, X₂ = CH₃, Z = OCH₂, p = 1, q = 1, and the molecular weight is approximately 20000, wherein the value of n₁, n₂, n₃ are the same as that in compound H1-1. 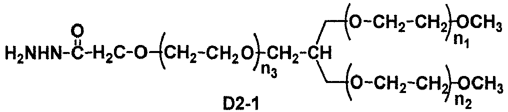
A: Into a dry and clean 1 L round-bottom flask fitted with a nitrogen-introducing tube, 0.32 g of sodium hydride (60 wt%, in oil) was added. With introducing nitrogen thereinto, 400 mL anhydrous tetrahydrofuran was added, and then 40 g of branched PEG obtained in example 1 (H1-1 treated by azeotropic removal of water with toluene) in tetrahydrofuran was added dropwisely in an ice-bath, followed by 3 hours of stirring at room temperature. Then, 2.2 mL ethyl bromoacetate was added, followed by 24 hours of reaction at room temperature. The reaction was quenched by a small amount of saturated ammonium chloride solution. The liquid was concentrated, and 400 mL of dichoromethane was added. The resulting product was washed with saturated salt solution (100 mL thrice), dried, concentrated again, and recrystallized to obtain the white branched PEG ester intermediate (D2'). 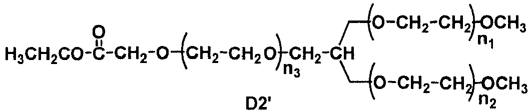
   ¹H NMR data of the intermediate D2' are as follows:
   ¹H NMR (CDCl₃) δ (ppm): 1.31 (-COOCH₂C***H***₃), 2.51 (-C***H***(CH₂)₃-), 3.35 (C***H**₃*O-), 3.40-3.80 (-C***H***₂C***H***₂O-, -CHC***H***₂O-, -OC***H***₂CH₃), 4.33 (-OC***H***₂COO-); Mₙ = 20000, PDI = 1.05.
B, Into a dry and clean 500 mL round-bottom flask, 40 g of branched PEG ester intermediate obtained in step A (D2') and 200 mL of 80% hydrazine hydrate were added in sequence. The whole was stirred till dissolution. Thereafter, the reaction was conducted at room temperature for 24 hours. After completion of the reaction, 200 mL deionized water was added, the solution was extracted with dichloromethane (100 mL) thrice. The organic phase was collected, washed with saturated salt solutions, dried, filtrated, concentrated, and recrystallized to obtain the hydrazide derivative (D2-1).
   ¹H NMR data of the hydrazide derivative D2-1 are as follows:
   ¹H NMR (CDCl₃) δ (ppm): 2.21 (-OCH₂CONH₂N***H***₂), 2.51 (-C***H***(CH₂)₃-), 3.35 (C***H**₃*O-), 3.40-3.80 (-C***H***₂C***H***₂O-, -CHC***H**₂*O-), 4.26 (-C***H***₂CONH₂), 7.52 (-CH₂CON***H***₂NH₂); Mₙ = 20000, PDI = 1.05.

### Synthesis of amide derivative D1-1

In the case of synthesizing the amide derivative (D1-1), R = OCH₂CONH₂, L₁ = CH₂, L₂ = CH₂, R₁ = H, X₁ = CH₃, X₂ = CH₃, Z = OCH₂, p = 1, q = 1, and the molecular weight is approximately 20000, wherein the value of n₁, n₂, n₃ are the same as that in compound H1-1.

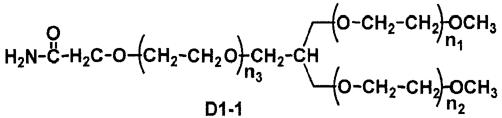

Into a dry and clean 500 mL high-pressure reactor, 40 g of branched PEG ester intermediate obtained in example 4-4 step A (D2') was added and then 200 mL of 34% ammonia water was added. The whole was stirred until dissolution, followed by 24 hours of reaction at 80°C. Thereafter, 200 mL deionized water was added, followed by extraction with dichloromethane (100 mL thrice). The organic phase was collected, washed with saturated salt solutions, dried, filtrated, concentrated, and recrystallized to obtain the white amide compounds (D1-1).
¹H NMR data of the amide compound D1-1 are as follows:
¹H NMR (CDCl₃) δ (ppm): 2.51 (C***H***(CH₂)₃), 3.35 (C***H**₃*O-), 3.40-3.80 (-C***H**₂*C***H**₂*O*-,* -CHC***H***₂O-) , 4.26 (-OC***H***₂CONH₂), 5.52 (-CH₂CON***H***₂); Mₙ = 20000, PDI = 1.05.

### Synthesis of carboxylic acid derivative D4-1

In the case of synthesizing the carboxylic acid derivative (D4-1), R = OCH₂COOH, L₁ = CH₂, L₂ = CH₂, R₁ = H, X₁ = CH₃, X₂ = CH₃, Z = OCH₂, p = 1, q = 1, and the molecular weight is approximately 20000, wherein the value of n₁, n₂, n₃ are the same as that in compound H1-1.

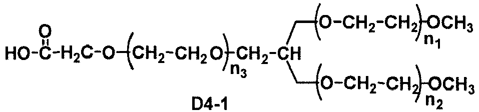

Into a dry and clean 500 mL high pressure reactor, 40 g of branched PEG ester intermediate obtained in example 4-4 step A (D2') was added, and then 200 mL of 1 mol/L aqueous sodium hydroxide was added. The whole was stirred until dissolution followed by 24 hours of reaction at 80°C. In an ice-bath, the solution was adjusted to pH 3 with hydrochloric acid (3 mol/L). The aqueous phase was extracted with dichloromethane (100 mL thrice). The organic phase was collected, washed with saturated salt solutions, dried, filtrated, concentrated, and recrystallized to obtain the white carboxylic acid derivative (D4-1).
¹H NMR data of the carboxylic acid compound D4-1 are as follows:
¹H NMR (CDCl₃) δ (ppm): 2.51 (-C***H***(CH₂)₃-), 3.35 (C***H**₃*O-), 3.40-3.80 (-C***H**₂*C***H**₂*O*-,* -CHC***H***₂O-), 4.31 (-OC***H***₂COOH); Mₙ = 20000, PDI = 1.05.

### Example 5

### Synthesis of α,β-unsaturated ester compound E2-1

In the case of synthesizing the α,β-unsaturated ester compound (E2-1), 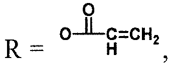 L₁ = CH₂, L₂ = CH₂, R₁ = H, X₁ = CH₃, X₂ = CH₃, Z = OCH₂CH₂O, p = 1, q = 1, and the molecular weight is approximately 30000, wherein the value of n₁, n₂, n₃ are the same as that in compound H1-3.

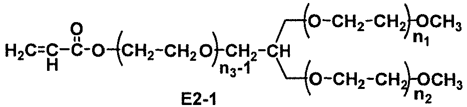

Into a dry and clean 1 L round-bottom flask fitted with a nitrogen-introducing tube, 40 g of branched PEG obtained in example 1 (H1-1 treated by azeotropic removal of water with toluene) was added. With introducing nitrogen thereinto, 600 mL of anhydrous and oxygen-free tetrahydrofuran was added. The whole was stirred at room temperature until dissolution. In an ice-bath, 10 mL of triethylamine and 2 mL of acryloyl chloride were added in sequence followed by 24 hours of reaction at room temperature. The liquid was concentrated, and 200 mL deionized water was added followed by extraction with dichloromethane (3 × 75 mL). The organic phase was collected, washed with saturated sodium chloride (3 × 50 mL), dried, concentrated again, and recrystallized to obtain the product (E2-1) in white solid state.
¹H NMR data of the α,β-unsaturated ester compound E2-1 are as follows:
¹H NMR (CDCl₃) δ (ppm): 2.51 (-C***H***(CH₂)₃-), 3.35 (*C**H**₃*O-), 3.40-3.80 (-C***H**₂*C***H**₂*O*-,* -CHC***H***₂O-, -OC***H***₂CH₂OCO-), 4.28 (-CH₂C***H***₂OCO-), 5.60-6.31 (C***H***₂=C***H***COO-); Mₙ = 30000, PDI= 1.10.

### Synthesis of propenyl ether derivative F2-1

In the case of synthesizing the propenyl ether derivative (F2-1), R = OCH₂CH=CH₂, L₁ = CH₂, L₂ = CH₂, R₁ = H, X₁ = CH₃, X₂ = CH₃, Z = OCH₂CH₂O, p = 1, q = 1, and the molecular weight is approximately 30000, wherein the value of n₁, n₂, n₃ are the same as that in compound H1-3.

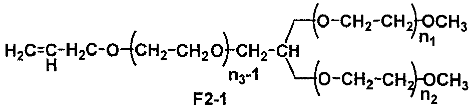

Into a dry and clean 1 L round-bottom flask fitted with a nitrogen-introducing tube, 0.32 g of sodium hydride (60 wt%, in oil) was added. With introducing nitrogen thereinto, 400 mL of anhydrous tetrahydrofuran was added. The tetrahydrofuran solution of 40 g of branched PEG obtained in example 1 (H1-3 treated by azeotropic removal of water with toluene) was added dropwisely in an ice-bath. After stirring at room temperature for 3 hours, 2 mL 3-bromopropene was added thereto, followed by 24 hours of reaction at room temperature. The reaction was quenched by a small amount of saturated ammonium chloride solution. The liquid was concentrated, and 200 mL of dichoromethane was added. The resulting product was washed with saturated sodium chloride (3 × 50 mL), dried, concentrated again, and recrystallized to obtain the propenyl ether derivative (F2-1) in white solid state.
¹H NMR data of propenyl ether derivative (F2-1) are as follows:
¹H NMR (CDCl₃) δ (ppm): 2.51 (-C***H***(CH₂)₃-), 3.35 (C***H**₃*O-), 3.40-3.80 (-C***H**₂*C***H**₂*O*-,* -CHC***H***₂O-), 4.05 (-OC***H***₂CH=CH₂), 5.31-6.06 (-OCH₂C***H***=CH₂); Mₙ = 30000, PDI = 1.10.

### Synthesis of glycidyl ether derivative F4-1

In the case of synthesizing the glycidyl ether derivative (F4-1), 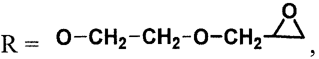 L₁ = CH₂, L₂ = CH₂, R₁ = H, X₁ = CH₃, X₂= CH₃, Z = OCH₂CH₂, p = 1, q = 1, and the molecular weight is approximately 30000, wherein the value of n₁, n₂, n₃ are the same as that in compound H1-3.

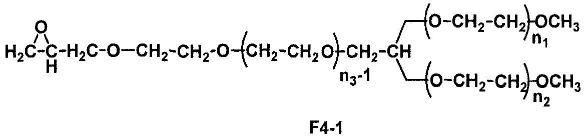

Into a dry and clean 1 L round-bottom flask fitted with a nitrogen-introducing tube, 0.32 g of sodium hydride (60 wt%, in oil) was added. With introducing nitrogen thereinto, 400 mL of anhydrous tetrahydrofuran was added, and the tetrahydrofuran solution of 40 g of branched PEG obtained in example 1 (H1-3 treated by azeotropic removal of water with toluene) was added dropwisely in an ice-bath. After the whole was stirred at room temperature for 3 hours, 2 mL of epichlorohydrin was added followed by 24 hours of reaction at room temperature. The reaction was quenched by a small amount of saturated ammonium chloride solution. The liquid was concentrated, and 200 mL of dichoromethane was added. The resulting product was washed with saturated sodium chloride (3 × 50 mL), dried, concentrated again, and recrystallized to obtain the epoxy derivative (F4-1) in white solid state.
¹H NMR data of glycidyl ether derivative (F4-1) are as follows:
¹H NMR (CDCl₃) δ (ppm) : 2.38 (-CH₂C***H***(O)CH₂O-), 2.51 (-C***H***(CH₂)₃), 2.63 (-CH₂C***H***(O)CH₂O-), 3.35 (C***H**₃*O-), 3.40-3.80 (-C***H**₂*C***H**₂*O*-,* -CHC***H***₂O-, -C***H***₂CH (O)CH₂O-); Mₙ = 30000, PDI = 1.10.

### Example 6

### Synthesis of active alkyne compound G2-1

In the case of synthesizing the active alkyne compound (G2-1), L₁ = CH₂, L₂= CH₂, R₁ = H, X₁ = CH₃, X₂ = CH₃, Z = OCH₂COO, p = 1, q = 1, and the molecular weight is approximately 20000, wherein the value of n₁, n₂, n₃ are the same as that in compound D4-1.

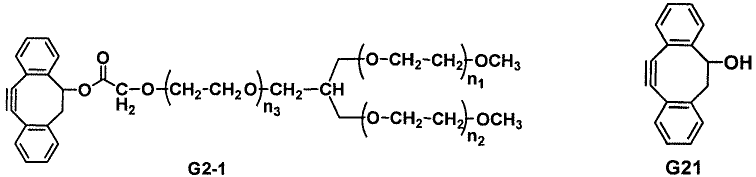

Into a dry and clean 1 L round-bottom flask fitted with a nitrogen-introducing tube, 40 g of branched PEG acetic acid derivative (D4-1 treated by azeotropic removal of water with toluene), 20 mL of triethylamine and 10 g of alcohol (G21) were added. With introducing nitrogen thereinto, 200 mL of dichloromethane was added. The whole was stirred until dissolution. Thereafter, 20 g of dicyclohexylcarbodiimide (DCC) was added, followed by 24 hours of reaction at room temperature. The resulting product was filtrated to remove undissolved substances, concentrated, and recrystallized from isopropanol to obtain the active alkyne compound (G2-1) in white solid state.
¹H NMR data of the active alkyne compound G2-1 are as follows:
¹H NMR (CDCl₃) δ (ppm): 2.51 (-C***H***(CH₂)₃-), 2.91-3.15 (PhC***H***₂CH-), 3.35 (C***H**₃*O-), 3.40-3.80 (-C***H**₂*C***H**₂*O*-,* PhC***H***₂CH(O)C***H***₂-), 4.53 (-OC***H***₂COO-), 7.32-7.54 (C₆***H***₄-); Mₙ = 20000, PDI = 1.05.

### Example 7

### Synthesis of aldehyde derivative D5-1

In the case of synthesizing the aldehyde derivative (D5-1), R = OCH₂CHO, L₁ = CH₂, L₂ = CH₂, R₁ = H, X₁ = CH₃, X₂ = CH₃, Z = OCH₂, p = 1, q = 1, and the molecular weight is approximately 20000, wherein the value of n₁, n₂, n₃ are the same as that in compound H1-1.

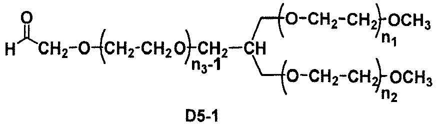

Into a dry and clean 500 mL round-bottom flask fitted with a nitrogen-introducing tube, 40 g of branched PEG obtained in example 1 (H1-1 treated by azeotropic removal of water with toluene) was added. With introducing nitrogen thereinto, 100 mL anhydrous and oxygen-free dichloromethane, 100 mL of dimethyl sulfoxide and 1 mL of pyridine were added in sequence in an ice-bath. Thereafter, 0.88 mL of trifluoroacetic acid was added dropwisely, followed by 1 hour of stirring in an ice-bath. Then, 5 g of dicyclohexylcarbodiimide (DCC) in dichloromethane was added dropwisely, followed by 24 hours of stirring at room temperature. The liquid was filtrated to remove undissolved substances, added with 200 mL of dichloromethane, and washed with deionized water (3 × 100 mL) and saturated salt solutions in sequence. The organic phases was collected, washed with saturated salt solutions (3 × 100 mL), dried, concentrated, and recrystallized to obtain the aldehyde derivative (D5-1) in white solid state.
¹H NMR data of aldehyde derivative D5-1 are as follows:
¹H NMR (CDCl₃) δ (ppm): 2.51 (-C***H***(CH₂)₃-), 3.35 (C***H**₃*O-), 3.40-3.80 (-C***H**₂*C***H**₂*O*-,* -CHC***H***₂O-), 4.23 C-OC***H***₂CHO) ,9.80 (-OCH₂C***H***O); Mₙ = 20000, PDI = 1.05.

### Synthesis of propionaldehyde derivative D5-2

In the case of synthesizing the propionaldehyde derivative (D5-2), R = OCH₂CH₂ CHO, L₁ = CH₂, L₂ = CH₂, R₁ = H, X₁ = CH₃, X₂ = CH₃, Z = OCH₂CH₂, p = 1, q = 1, and the molecular weight is approximately 20000, wherein the value of n₁, n₂, n₃ are the same as that in compound H1-1. 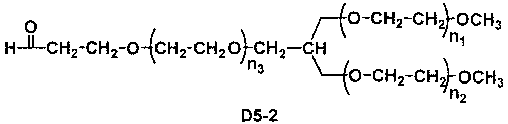
A, Into a dry and clean 1 L round-bottom flask fitted with a nitrogen-introducing tube, 40 g of branched PEG obtained in example 1 (H1-1) and 5 g of sodium hydroxide were added. With introducing nitrogen thereinto, 400 mL of toluene was added, and then 2 mL of 2-(2-bromoethyl)-1,3-dioxane was added dropwisely. The whole was heated until reflux followed by 24 hours of reaction under reflux. After completion of the reaction, 400 mL deionized water was added, then the aqueous layer was extracted with dichloromethane (3 × 200 mL). The organic phase was collected, washed with saturated salt solutions (3 × 100 mL), dried, concentrated, and recrystallized to obtain the white branched PEG acetal intermediate (D5'). 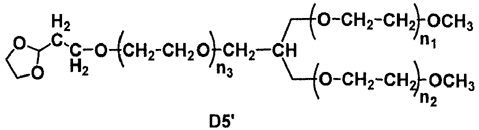
   ¹H NMR data of the PEG acetal intermediate D5' are as follows:
   ¹H NMR (CDCl₃) δ (ppm): 1.91 (-OC***H***₂CH₂CHO(O)-) , 2.51 (-C***H***(CH₂)₃-), 3.35 (C***H**₃*O-), 3.40-3.90 (-OC***H**₂*C***H**₂*O-, -CHC***H***₂O-, -OC***H***₂CH₂CHO(O)-), 4.89 (-OCH₂CH₂C***H***O(O)-).
B, Into a dry and clean 1 L round-bottom flask, 40 g of branched PEG acetal intermediate obtained in step A was added, and then 400 mL of deionized water was added. The whole was stirred until dissolution. The pH was adjusted to 1.0 with hydrochloric acid (1 mol/L) in an ice-bath, followed by 4 hours of reaction at room temperature. The liquid was extracted with dichloromethane (3 × 200 mL). The organic phase was collected, washed with saturated salt solutions, dried, filtrated, concentrate, and recrystallized to obtain the white PEG propionaldehyde derivative (D5-2).
   ¹H NMR data of PEG propionaldehyde derivative D5-2 are as follows:
   ¹H NMR (CDCl₃) δ (ppm): 2.51 (-C***H***(CH₂)₃), 2.63 (-OCH₂C***H***₂CHO) 3.35 (C***H**₃*O-), 3.40-3.80 (-C***H**₂*C***H**₂*O*-,* -CHC***H***₂O-, -OC***H***₂CH₂CHO), 9.75 (-OCH₂CH₂C***H***O); Mₙ = 20000, PDI = 1.05.

### Example 8

### Synthesis of maleimide derivative El-1

In the case of synthesizing the maleimide derivative (E1-1), 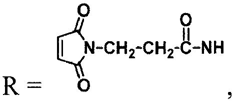 L₁ = CH₂, L₂ = CH₂, R₁ = H, X₁ = CH₃, X₂= CH₃, Z = NHCOCH₂CH₂, p = 1, q = 1, and the molecular weight is approximately 20000, wherein the value of n₁, n₂, n₃ are the same as that in compound C3-1.

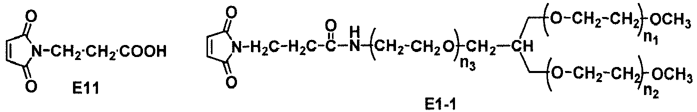

Into a dry and clean 1 L round-bottom flask fitted with a nitrogen-introducing tube, 40 g of branched PEG amine derivative obtained in example 4 (C3-1 treated by azeotropic removal of water with toluene) and 10 g of maleimide propionic acid (E11) were added. With introducing nitrogen thereinto, dichloromethane as solvent (600 mL) was added, and then the whole was stirred until dissolution. Thereafter, 20 mL triethylamine and 20 g of dicyclohexylcarbodiimide (DCC) were added in sequence, followed by 24 hours of reaction at room temperature. The resulting product was filtrated to remove undissolved substances, concentrated, and recrystallized from isopropanol to obtain the white maleimide derivative (E1-1).
¹H NMR data of maleimide derivative E1-1 are as follows:
¹H NMR (CDCl₃) δ (ppm): 2.51 (-C***H***(CH₂)₃), 2.70 (-NHCOC***H***₂CH₂-), 3.35 (C***H**₃*O-), 3.40-3.80 (-C***H**₂*C***H**₂*O*-,* -CHC***H***₂O-, -NHCOC***H***₂CH₂N-), 3.92 (-NHCOCH₂C***H***₂N-), 6.81 (-C***H***=C***H***-); Mn = 20000, PDI = 1.05.

### Example 9: Preparation method of paclitaxel modified with acetic acid derivative (D4-1)

Into a dry and clean 250 mL round-bottom flask fitted with a nitrogen-introducing tube, 1.8 g of branched PEG acetic acid derivative prepared in example 4 (D4-1 with a molecular weight of approximate 20000 and treated by azeotropic removal of water using toluene), 90 mg of paclitaxel and 12 mg of DMAP were added. With introducing nitrogen thereinto, dichloromethane as solvent (50 mL) was added, and then the whole was stirred until dissolution. Thereafter, 30 mg of dicyclohexylcarbodiimide (DCC) in dicholomethane was added, followed by 24 hours of reaction at room temperature. The resulting product was filtered to remove undissolved substances, concentrated and precipitated with diethyl ether to obtain pegylated paclitaxel. The yield is 1.7 g (87%).

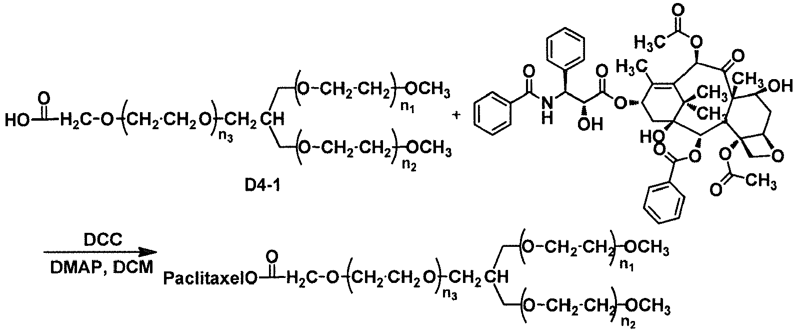

### Example 10: Preparation method of β-interferon modified with PEG succinimide derivative (A1-2)

Into a dry and clean 50 mL round-bottom flask fitted with a nitrogen-introducing tube, 60 mg of branched PEG succinimide derivative prepared in example 2-3 (A1-2, molecular weight of 20000) was added. With introducing nitrogen thereinto, 7.5 mL of PBS buffer salt solution (pH = 8.0) plus *β*-interferon (1 g/L) was added, shaken for 7 hours at 25 °C, and shaken again for 24 hours at 4 °C. Subsequently the concentration of *β*-interferon was diluted to 0.5 g/L with 7.5 mL of PBS buffer salt solution (pH = 8.0). The resulting product was purified by agarose column chromatography, and then mono-substituted and bi-substituted components were collected respectively. Thereafter, an ultrafiltration concentration operation was carried out. The results of SDS-PAGE showed that there was no free *β*-interferon in the final product, and the results of GPC showed that there was no free PEG molecule either.

### Example11: Preparation method of lysozyme modified with PEG maleimide derivative (E1-1)

Into a dry and clean 50 mL round-bottom flask, 10 mL of phosphate buffer solution (pH = 7.4) plus lysozyme (0.5 mmol/L) was added, shaken till all were dissolved followed by cooling to 4 °C. Thereafter, 2.5 molar equivalent of 2-iminothiolane hydrochloride was added followed by 24 hours of reaction. As a result, all the amino groups of lysozyme were converted into mercapto groups. After removing the excess 2-iminothiolane hydrochloride, 3 molar equivalents of branched PEG maleimide derivative prepared in example 8 (E1-1, molecular weight of 20000) was added, followed by 24 hours of reaction at 4 °C. The remaining inorganic salts were removed, and the product was purified by an ion exchange resin. The results of SDS-PAGE showed that there was no free lysozyme in the final product, and the results of GPC showed that there was no free PEG molecule either.

### Example 12: Preparation method of antisense oligodeoxynucleotide modified with PEG succinimide derivative (A1-2)

Into a dry and clean 50 mL round-bottom flask, 5'-amino-antisense oligodeoxynucleotide (1 mg, 152 mmol) and 10 mL of PBS buffer solution (pH = 7.0) were added and shaken until all were dissolved. Then, 3 molar equivalents of branched PEG succinimide derivative prepared in example 2 (A1-2, molecular weight of 20000) was added, followed by 4 hours of reaction at room temperature. Ultrafiltration was carried out using deionized water to remove unreacted PEGs and remaining inorganic salts. The final product was characterized by GPC, which showed no free PEG molecule in the final product.

## Claims

1. A monofunctional branched poly(ethylene glycol) (PEG) represented by the following general formula (1): 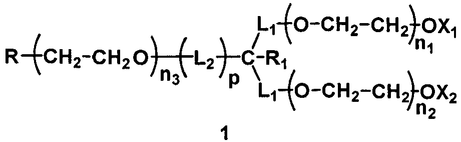
wherein X₁ and X₂ are each independently a hydrocarbon group having 1 to 20 carbon atoms at the terminal end of the two branch chains,
wherein n₁ and n₂ are each independently a value selected from 1 to 1000,
wherein n₃ is a value selected from 11 to 1000,
wherein R₁ is a hydrogen atom, a hydrocarbon group having 1 to 20 carbon atoms, or a hydrocarbon group having 1 to 20 carbon atoms with one or more groups selected from a group consisting of an amide bond, an ether bond, a double bond, a triple bond and a tertiary amino group, and
wherein R is a functional group on the terminal of the main chain of the branched poly(ethylene glycol), and R can react with the reactive group of a bio-related substance,
wherein p is 0 or 1,
wherein L₁ and L₂ are each independently a linking group and the combination of L₁ and L₂ is selected from one of a group consisting of:
group I: L₁ is a divalent hydrocarbon group having 1 to 20 carbon atoms, and L₁ excludes a methylene group, when p is 0,
group II: L₁ is a divalent hydrocarbon group having 1 to 20 carbon atoms and containing at least one of a group consisting of an ether bond, a thioether bond, a double bond, a triple bond, and an amino group, when p is 0,
group III: L₁ and L₂ are each independently a divalent hydrocarbon group having 1 to 20 carbon atoms, when p is 1,
group IV: L₁ is a divalent hydrocarbon group having 1 to 20 carbon atoms, excluding a methylene group, and wherein L₂ is a divalent hydrocarbon group having 1 to 20 carbon atoms containing an ether bond, when p is 1,
group V: L₂ is a divalent hydrocarbon group having 1 to 20 carbon atoms, and wherein L₁ is a divalent hydrocarbon group having 1 to 20 carbon atoms containing an ether bond, when p is 1,
and group VI: L₁ and L₂ are each independently a divalent hydrocarbon group having 1 to 20 carbon atoms and containing at least one of a group consisting of an ether bond, a thioether bond, a double bond, a triple bond, and an amino group, when p is 1, wherein L₁ excludes an ether bond when L₂ contains an ether bond, and wherein L₂ excludes an ether bond when L₁ contains an ether bond,
wherein said monofunctional branched poly(ethylene glycol) is obtainable by a process in which 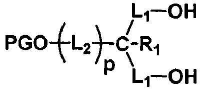 is a starting initiator for a first polymerization, and wherein 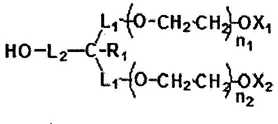 is an intermediate for a second polymerization, and
PG is a protective group of a hydroxyl group.

2. The monofunctional branched PEG according to claim 1, wherein X₁ and X₂ are each independently a group selected from the group consisting of a methyl, an ethyl, a propyl, a propenyl, a propinyl, an isopropyl, a butyl, a tertiary butyl, a pentyl, a heptyl, a 2-ethylhexyl, an octyl, a nonyl, a decyl, an undecyl, a dodecyl, a tridecyl, a tetradecyl, a pentadecyl, a hexadecyl, a heptadecyl, an octodecyl, a nonadecyl, an eicosyl, a benzyl and a butylphenyl, and X₁ and X₂ are the same or different from each other in one molecule.

3. The monofunctional branched PEG according to claim 1, wherein R is selected from the following groups: 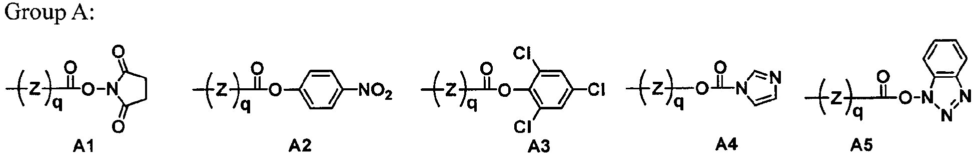 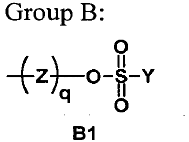 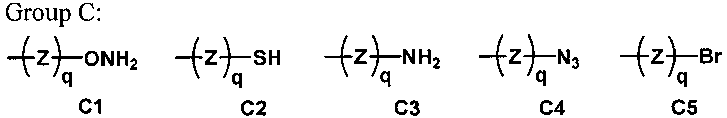 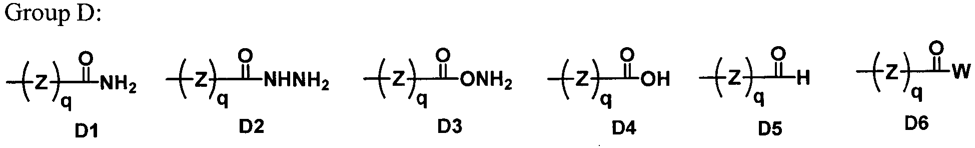 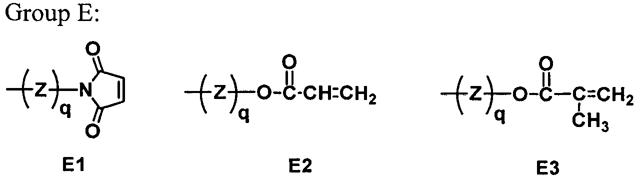 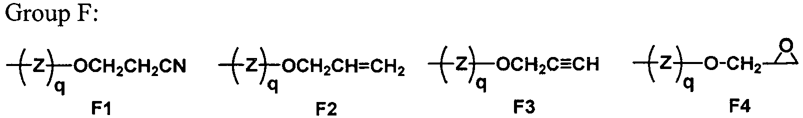 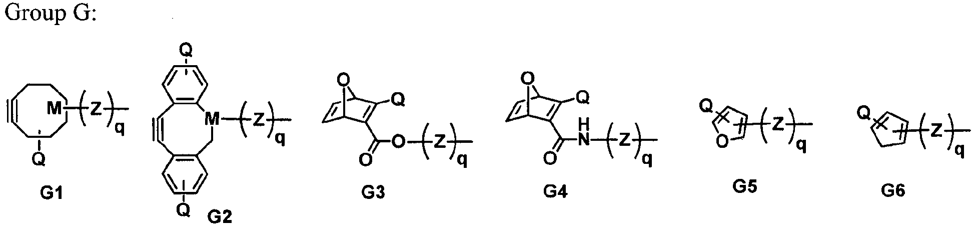 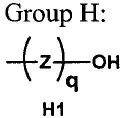 wherein Z is an alkylene group or an alkylene group containing at least one group selected from a group consisting of an amide bond, an ether bond, a double bond, a triple bond and a secondary amino group,
wherein q is 0 or 1,
wherein Y is a hydrocarbon group having 1 to 10 carbon atoms or a hydrocarbon group having 1 to 10 carbon atoms which contains a fluorine atom,
wherein Q is hydrogen or a group favoring inductive effect, conjugative effect or both of electrons on a unsaturated bond,
wherein M is a carbon atom or a nitrogen atom on the ring, and
wherein W is a halogen atom.

4. The monofunctional branched PEG according to claim 3, wherein Z is selected from a group consisting of: a methylene group, an 1,2-ethylene group, a 1,3-propylene group, a 1,2-propylene group, an isopropylene group, a butylene group, a pentylene group and a hexylene group.

5. The monofunctional branched PEG according to claim 1, wherein each of n₁ and n₂ is independently a value selected from 10 to 800, preferably from 25 to 800, and more preferably from 50 to 500.

6. The monofunctional branched PEG according to claim 1, wherein n₃ is a value selected from 11 to 800, preferably from 11 to 500, and more preferably from 11 to 200.

7. The monofunctional branched PEG according to claim 1, wherein the bio-related substance is selected from a group consisting of polypeptide, protein, enzyme, small molecule drug, dye, liposome, nucleoside, nucleotide, oligonucleotide, polynucleotide, nucleic acid, polysaccharide, steroid, lipid, phospholipid, glycolipid, glycoprotein, cell and virus.

8. The monofunctional branched PEG according to claim 1, wherein the reactive group of a bio-related substance is selected from one of a group consisting of an amino group, a mercapto group, an unsaturated bond, a carboxyl group and a hydroxyl group.

9. A production method of the monofunctional branched PEG according to any one of claims 1-8, including steps as follows:
Step (a): In a coinitiator system consisting of a small molecule initiator (4) and a base, ethylene oxide is polymerized to the two geometrically symmetrical hydroxyl groups of initiator (4) to generate two branch chains; Thereafter, the terminal ends of the two newly formed branch chains are deprotonated to obtain an intermediate (5),
Step (b): The initiating active terminals of the two branch chains of intermediate (5) are alkyl-etherified to obtain an intermediate (6);
Step c): The terminal hydroxyl group on the symmetry axis of intermediate (6) is deprotected to obtain an intermediate (7);
Step (d): Ethylene oxide is polymerized to the deprotected terminal hydroxyl group on the symmetry axis of intermediate (7) to generate a main chain, which is subsequently protonated to obtain an intermediate (3) with a terminal hydroxyl group;
Step (e): The terminal of the main chain of intermediate (3) is functionalized, and thereby the monofunctional branched poly(ethylene glycol) of the general formula (1) can be obtained; 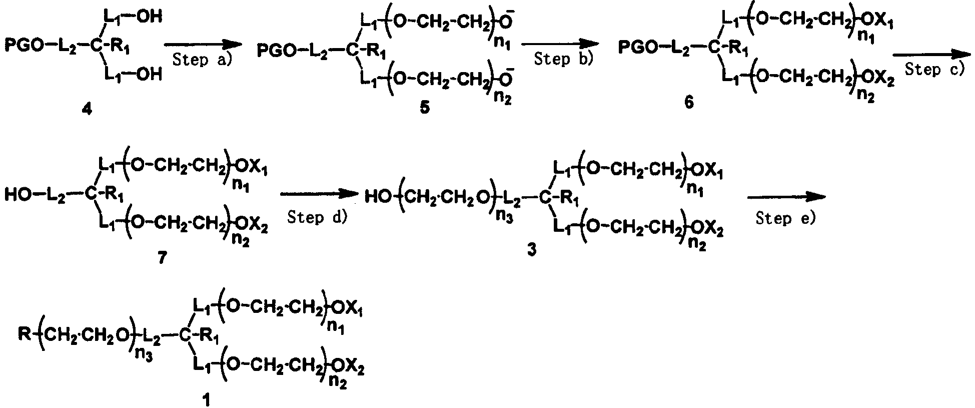 wherein PG represents a protective group of a hydroxyl group.

10. A bio-related substance modified by the monofunctional branched PEG according to any one of the claims 1 to 8, and having a chemical structure shown in the general formula (2): 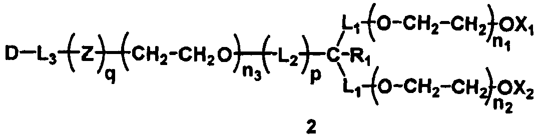 wherein D is a bio-related substance, q is 0 or 1, and
wherein Z is a linking group through which a functional group capable of reacting with the bio-related substance is linked to the main chain of PEG, and can react with the bio-related substance to form a residue group L₃.

11. The PEG-modified bio-related substance according to claim 10, wherein D is selected from a group consisting of polypeptide, protein, enzyme, small molecule drug, dye, liposome, nucleoside, nucleotide, oligonucleotide, polynucleotide, nucleic acid, polysaccharide, steroid, lipid, phospholipid, glycolipid, glycoprotein, cell and virus.

12. The PEG-modified bio-related substance according to claim 11, wherein D is selected from a group consisting of polypeptide, protein, enzyme, dye, liposome, nucleoside, nucleotide, oligonucleotide, polynucleotide, nucleic acid, polysaccharide, steroid, lipid, phospholipid, glycolipid, glycoprotein, cell and virus, preferably selected from a group consisting of polypeptide, protein, enzyme, nucleoside, nucleotide, oligonucleotide, polynucleotide and nucleic acid, and more preferably selected from a group consisting of interferon, lysozyme and antisense oligodeoxynucleotide.

13. The PEG-modified bio-related substance according to claim 10, wherein L₃ is selected from a group consisting of a triazole bond, an isoxazole bond, an ether bond, an amide bond, an imide bond, an imino group, a secondary amino group, a tertiary amino group, a thioester bond, a disulfide bond, a urethane bond, a thiocarbonate bond, a sulfonate bond, a sulfamide bond, a carbamate bond, a tyrosine group, a cysteine group, a histidine group and the combination thereof.

14. A compound of the following formula (3) 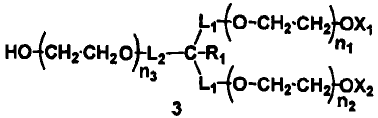 wherein X₁, X₂, n₁, n₂, n₃, L₁, L₂ and R₁ are the same as those defined in general formula (1) of claim 1.

15. A production method of the intermediate compound having the formula (3) below, which including steps as follows:
Step (a): in a coinitiator system consisting of a small molecule initiator (4) and a base, ethylene oxide is polymerized to the two geometrically symmetrical hydroxyl groups of initiator (4) to generate two branch chains; thereafter, the terminal ends of the two newly formed branch chains are deprotonated to obtain an intermediate (5),
Step (b): the initiating active terminals of the two branch chains of intermediate (5) are alkyl-etherified to obtain an intermediate (6);
Step (c): the terminal hydroxyl group on the symmetry axis of intermediate (6) is deprotected to obtain an intermediate (7);
Step (d): ethylene oxide is polymerized to the deprotected terminal hydroxyl group on the symmetry axis of intermediate (7) to generate a main chain, which is subsequently protonated to obtain an intermediate (3) with a terminal hydroxyl group; 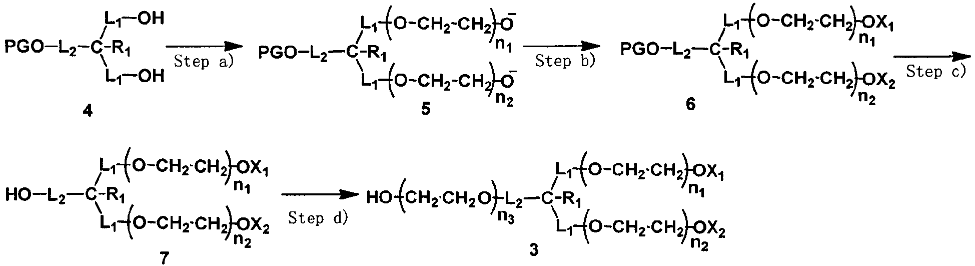 wherein PG represents a protective group of a hydroxyl group.

## Patentansprüche

1. Monofunktionelles, verzweigtes Poly(ethylenglykol) (PEG), dargestellt durch die folgende Formel (1): 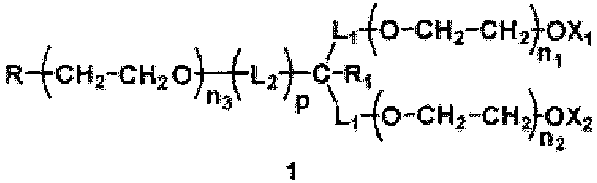 worin X₁ und X₂ jeweils unabhängig eine Kohlenwasserstoff-Gruppe mit 1 bis 20 Kohlenstoffatomen an dem terminalen Ende mit den zwei Verzweigungsketten sind,
worin n₁ und n₂ jeweils unabhängig ein Wert sind, ausgewählt aus 1 bis 1000,
worin n₃ ein Wert ist, ausgewählt aus 11 bis 1000,
worin R₁ ein Wasserstoffatom, eine Kohlenwasserstoff-Gruppe mit 1 bis 20 Kohlenstoffatomen oder eine Kohlenwasserstoff-Gruppe mit 1 bis 20 Kohlenstoffatomen mit einer oder mehreren Gruppen ist, ausgewählt aus einer Gruppe, bestehend aus einer Amid-Bindung, Ether-Bindung, Doppelbindung, Dreifachbindung und einer tertiären Amino-Gruppe, und
worin R eine funktionelle Gruppe an dem Terminus der Hauptkette des verzweigten Poly(ethylenglykols) ist und R mit der reaktiven Gruppe einer bio-bezogenen Substanz reagieren kann,
worin p 0 oder 1 ist,
worin L₁ und L₂ jeweils unabhängig eine Bindegruppe sind und die Kombination von L₁ und L₂ aus einer Gruppe ausgewählt sind, bestehend aus:
Gruppe I: L₁ ist eine bivalente Kohlenwasserstoff-Gruppe mit 1 bis 20 Kohlenstoffatomen und L₁ schließt eine Methylen-Gruppe aus, wenn p 0 ist,
Gruppe II: L₁ ist eine bivalente Kohlenwasserstoff-Gruppe mit 1 bis 20 Kohlenstoffatomen und mit zumindest einer von einer Gruppe, bestehend aus einer Ether-Bindung, Thioether-Bindung, Doppelbindung, Dreifachbindung und Amino-Gruppe, wenn p 0 ist,
Gruppe III: L₁ und L₂ sind jeweils unabhängig eine bivalente Kohlenwasserstoff-Gruppe mit 1 bis 20 Kohlenstoffatomen, wenn p 1 ist,
Gruppe IV: L₁ ist eine bivalente Kohlenwasserstoff-Gruppe mit 1 bis 20 Kohlenstoffatomen ohne eine MethylGruppe, und worin L₂ eine bivalente Kohlenwasserstoff-Gruppe mit 1 bis 20 Kohlenstoffatomen, enthaltend eine Ether-Bindung, ist, wenn p 1 ist,
Gruppe V: L₂ ist eine bivalente Kohlenwasserstoff-Gruppe mit 1 bis 20 Kohlenstoffatomen und worin L₁ eine bivalente Kohlenwasserstoff-Gruppe mit 1 bis 20 Kohlenstoffatomen, enthaltend eine Ether-Bindung, ist, wenn p 1 ist,
und Gruppe VI: L₁ und L₂ sind jeweils unabhängig eine bivalente Kohlenwasserstoff-Gruppe mit 1 bis 20 Kohlenstoffatomen und mit zumindest einer von einer Gruppe, bestehend aus einer Ether-Bindung, Thioether-Bindung, Doppelbindung, Dreifachbindung und Amino-Gruppe, wenn p 1 ist, worin L₁ eine Ether-Bindung ausschließt, wenn L₂ eine Ether-Bindung enthält, und worin L₂ eine Ether-Bindung ausschließt, wenn L₁ eine Ether-Bindung enthält,
worin das monofunktionelle verzweigte Poly(ethylenglykol) erhältlich ist durch ein Verfahren, bei dem 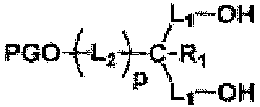
ein Ausgangsinitiator für eine erste Polymerisation ist und worin 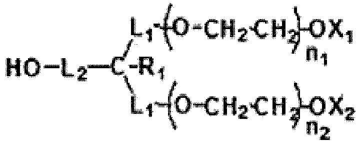
ein Zwischenprodukt für eine zweite Polymerisation ist und PG eine Schutzgruppe einer Hydroxyl-Gruppe ist.

2. Monofunktionelles, verzweigtes PEG nach Anspruch 1, worin X₁ und X₂ jeweils unabhängig eine Gruppe sind, ausgewählt aus der Gruppe bestehend aus Methyl, Ethyl, Propyl, Propenyl, Propinyl, Isopropyl, Butyl, tertiärem Butyl, Pentyl, Heptyl, 2-Ethylhexyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Benzyl und Butylphenyl und X₁ und X₂ gleich oder verschieden voneinander in einem Molekül sind.

3. Monofunktionelles verzweigtes PEG nach Anspruch 1, worin R ausgewählt ist aus den folgenden Gruppen: 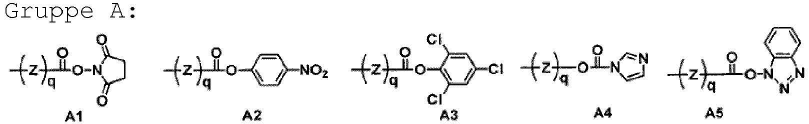 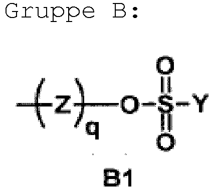 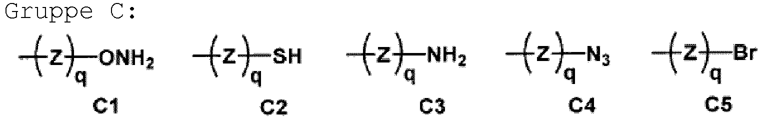 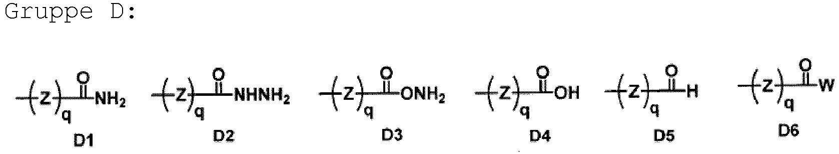 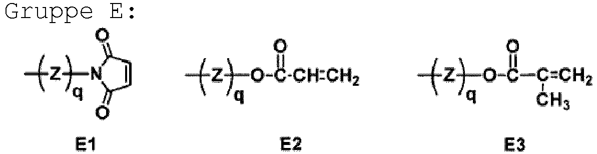 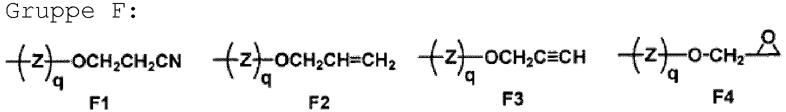 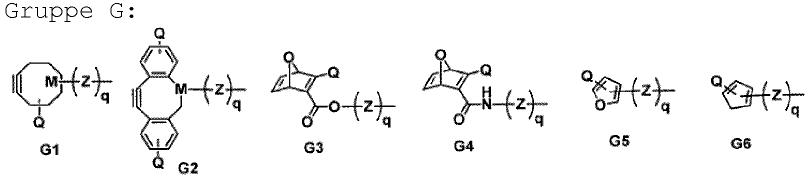 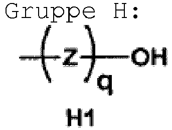 worin Z eine Alkylen-Gruppe oder Alkylen-Gruppe mit zumindest einer Gruppe ist, ausgewählt aus der Gruppe bestehend aus einer Amid-Bindung, Ether-Bindung, Doppelbindung, Dreifachbindung und sekundären Amino-Gruppe,
worin q 0 oder 1 ist,
worin Y eine Kohlenwasserstoff-Gruppe mit 1 bis 10 Kohlenstoffatomen oder eine Kohlenwasserstoff-Gruppe mit 1 bis 10 Kohlenstoffatomen ist, die ein Fluor-Atom enthält,
worin Q Wasserstoff oder eine Gruppe ist, die eine induktive Wirkung, konjugative Wirkung oder beides von Elektronen an einer ungesättigten Bindung favorisiert,
worin M ein Kohlenstoffatom oder Stickstoffatom am Ring ist und
worin W ein Halogenatom ist.

4. Monofunktionelles, verzweigtes PEG nach Anspruch 3, worin Z ausgewählt ist aus der Gruppe bestehend aus: Methylen-Gruppe, 1,2-Ethylen-Gruppe, 1,3-Propylen-Gruppe, 1,2-Propylen-Gruppe, Isopropylen-Gruppe, Butylen-Gruppe, Pentylen-Gruppe und Hexylen-Gruppe.

5. Monofunktionelles, verzweigtes PEG nach Anspruch 1, worin jedes von n₁ und n₂ unabhängig ein Wert ist, ausgewählt aus 10 bis 800, bevorzugt von 25 bis 800 und mehr bevorzugt von 50 bis 500.

6. Monofunktionelles, verzweigtes PEG nach Anspruch 1, worin n₃ ein Wert ist, ausgewählt aus 11 bis 800, bevorzugt 11 bis 500 und mehr bevorzugt 11 bis 200.

7. Monofunktionelles, verzweigtes PEG nach Anspruch 1, worin die bio-bezogene Substanz ausgewählt ist aus einer Gruppe bestehend aus Polypeptid, Protein, Enzym, klein-moleküligem Arzneimittel, Farbstoff, Liposom, Nukleosid, Nukleotid, Oligonukleotid, Polynukleotid, Nukleinsäure, Polysaccharid, Steroid, Lipid, Phospholipid, Glycolipid, Glycoprotein, Zellen und Virus.

8. Monofunktionelles, verzweigtes PEG nach Anspruch 1, worin die reaktive Gruppe einer bio-bezogenen Substanz ausgewählt ist aus einer von einer Gruppe, bestehend aus einer Amino-Gruppe, Mercapto-Gruppe, ungesättigten Bindung, Carboxyl-Gruppe und Hydroxyl-Gruppe.

9. Produktionsverfahren des monofunktionellen, verzweigten PEG nach einem der Ansprüche 1 bis 8, umfassend die folgenden Schritte:
Schritt (a): in einem Co-Initiatorsystem, bestehend aus einem Klein-Molekül-Initiator (4) und einer Base wird Ethylenoxid an die zwei geometrisch-symmetrischen Hydroxyl-Gruppen des Initiators (4) polymerisiert, zum Generieren von zwei Verzweigungsketten; danach werden die terminalen Enden der zwei neu-gebildeten Verzweigungsketten deprotoniert, unter Erhalt eines Zwischenproduktes (5),
Schritt (b): die initiierenden aktiven Enden der zwei Verzweigungsketten des Zwischenproduktes (5) werden Alkyl-verethert, unter Erhalt eines Zwischenproduktes (6),
Schritt (c): bei der terminalen Hydroxyl-Gruppe an der Symmetrieachse des Zwischenproduktes (6) wird die Schutzgruppe abgespalten, unter Erhalt eines Zwischenproduktes (7),
Schritt (d): Ethylenoxid wird an die von der Schutzgruppe befreiten terminalen Hydroxyl-Gruppe an der Symmetrieachse des Zwischenproduktes (7) polymerisiert, zum Generieren einer Hauptkette, die anschließend protoniert wird, unter Erhalt eines Zwischenproduktes (3) mit einer terminalen Hydroxyl-Gruppe,
Schritt (e): das Ende der Hauptkette des Zwischenproduktes (3) wird funktionalisiert und hierdurch kann das monofunktionelle, verzweigte Poly(ethylenglykol) der allgemeinen Formel (1) erhalten werden 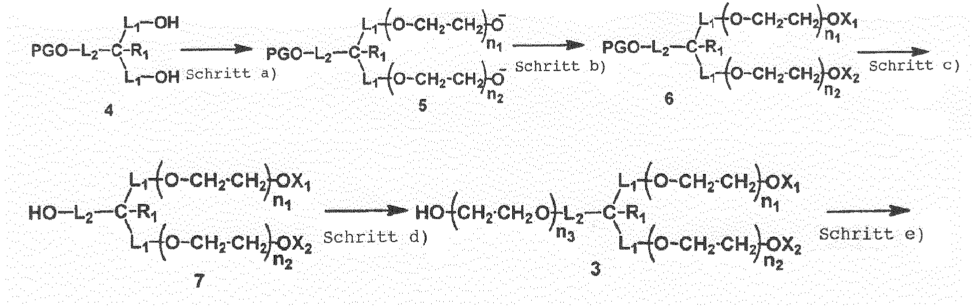 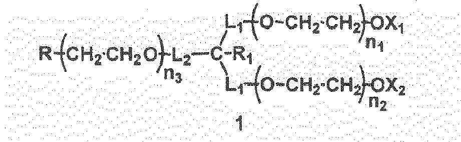 worin PG eine Schutzgruppe einer Hydroxyl-Gruppe ist.

10. Bio-bezogene Substanz, modifiziert durch das monofunktionelle, verzweigte PEG nach einem der Ansprüche 1 bis 8 und mit einer chemischen Struktur, dargestellt in der allgemeinen Formel (2): 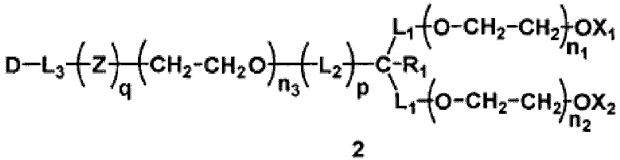 worin D eine bio-bezogene Substanz, q 0 oder 1 ist und
worin Z eine Bindegruppe ist, durch die eine funktionelle Gruppe, die mit der bio-bezogenen Substanz reagieren kann, an die Hauptkette von PEG gebunden ist und mit der bio-bezogenen Substanz zur Bildung einer Restgruppe L₃ reagieren kann.

11. PEG-modifizierte, bio-bezogene Substanz nach Anspruch 10, worin D ausgewählt ist aus einer Gruppe bestehend aus Polypeptid, Protein, Enzym, klein-moleküligem Arzneimittel, Farbstoff, Liposom, Nukleosid, Nukleotid, Oligonukleotid, Polynukleotid, Nukleinsäure, Polysaccharid, Steroid, Lipid, Phospholipid, Glycolipid, Glycoprotein, Zellen und Virus.

12. PEG-modifizierte, bio-bezogene Substanz nach Anspruch 11, worin D ausgewählt ist aus der Gruppe bestehend aus Polypeptid, Protein, Enzym, Farbstoff, Liposom, Nukleosid, Nukleotid, Oligonukleotid, Polynukleotid, Nukleinsäure, Polysaccharid, Steroid, Lipid, Phospholipid, Glycolipid, Glycoprotein, Zelle und Virus, bevorzugt ausgewählt aus einer Gruppe bestehend aus Polypeptid, Protein, Enzym, Nukleosid, Nukleotid, Oligonukleotid, Polynukleotid und Nukleinsäure und mehr bevorzugt ausgewählt aus einer Gruppe bestehend aus Interferon, Lysozym und Antisens-Oligodesoxynukleotid.

13. PEG-modifizierte, bio-bezogene Substanz nach Anspruch 10, worin L₃ ausgewählt ist aus der Gruppe bestehend aus einer Triazol-Bindung, Isoxazol-Bindung, Ether-Bindung, Amid-Bindung, Imid-Bindung, Imino-Gruppe, sekundären Amino-Gruppe, tertiären Amino-Gruppe, Thioester-Bindung, Disulfid-Bindung, Urethan-Bindung, Thiocarbonat-Bindung, Sulfonat-Bindung, Sulfamid-Bindung, Carbamat-Bindung, Tyrosin-Gruppe, Cystein-Gruppe, Histidin-Gruppe und der Kombination davon.

14. Verbindung der folgenden Formel (3) 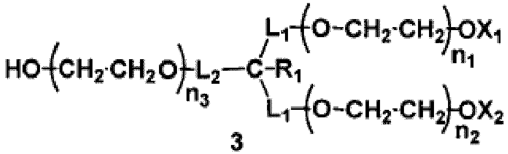 worin X₁, X₂, n₁, n₂, n₃, L₁, L₂ und R₁ gleich sind wie sie in der allgemeinen Formel (1) von Anspruch 1 definiert sind.

15. Produktionsverfahren der Zwischenprodukt-Verbindung mit der Formel (3) unten, umfassend die folgenden Schritte:
Schritt (a): in einem Co-Initiatorsystem, bestehend aus einem Klein-Molekül-Initiator (4) und einer Base wird Ethylenoxid an die zwei geometrisch-symmetrischen Hydroxyl-Gruppen des Initiators (4) polymerisiert, zum Generieren von zwei Verzweigungsketten; danach werden die terminalen Enden der zwei neu-gebildeten Verzweigungsketten deprotoniert, unter Erhalt eines Zwischenproduktes (5),
Schritt (b): die initiierenden aktiven Enden der zwei Verzweigungsketten des Zwischenproduktes (5) werden Alkyl-verethert, unter Erhalt eines Zwischenproduktes (6),
Schritt (c): bei der terminalen Hydroxyl-Gruppe an der Symmetrieachse des Zwischenproduktes (6) wird die Schutzgruppe abgespalten, unter Erhalt eines Zwischenproduktes (7),
Schritt (d): Ethylenoxid wird an die von der Schutzgruppe befreiten terminalen Hydroxyl-Gruppe an der Symmetrieachse des Zwischenproduktes (7) polymerisiert, zum Generieren einer Hauptkette, die anschließend protoniert wird, unter Erhalt eines Zwischenproduktes (3) mit einer terminalen Hydroxyl-Gruppe, 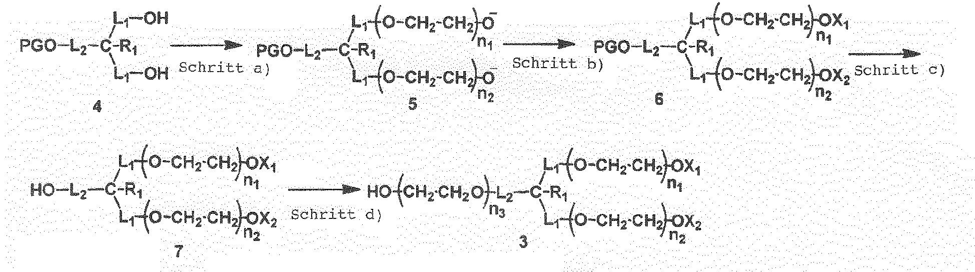 worin PG eine Schutzgruppe einer Hydroxyl-Gruppe ist.

## Revendications

1. Polyéthylène glycol (PEG) ramifié monofonctionnel représenté par la formule générale suivante (1) : 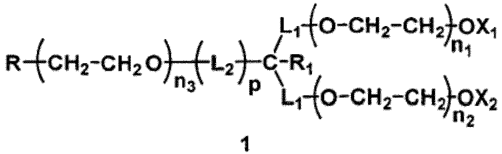
dans laquelle X₁ et X₂ sont chacun indépendamment un groupe hydrocarbure ayant 1 à 20 atomes de carbone à l'extrémité terminale des deux chaînes ramifiées,
dans laquelle n₁ et n₂ sont chacun indépendamment une valeur choisie de 1 à 1000,
dans laquelle n₃ est une valeur choisie de 11 à 1000,
dans laquelle R₁ est un atome d'hydrogène, un groupe hydrocarbure ayant 1 à 20 atomes de carbone, ou un groupe hydrocarbure ayant 1 à 20 atomes de carbone avec un ou plusieurs groupes choisis dans un groupe consistant en une liaison amide, une liaison éther, une liaison double, une liaison triple et un groupe amino tertiaire,
et
dans laquelle R est un groupe fonctionnel sur la terminaison de la chaîne principale du polyéthylène glycol ramifié, et R peut réagir avec le groupe réactif d'une substance bio-apparentée,
dans laquelle p est 0 ou 1,
dans laquelle L₁ et L₂ sont chacun indépendamment un groupe de liaison et la combinaison de L₁ et L₂ est choisie dans l'un d'un groupe consistant en :
groupe I : L₁ est un groupe hydrocarbure divalent ayant 1 à 20 atomes de carbone, et L₁ exclut un groupe méthylène, lorsque p est 0,
groupe II : L₁ est un groupe hydrocarbure divalent ayant 1 à 20 atomes de carbone et contenant au moins l'un d'un groupe consistant en une liaison éther, une liaison thioéther, une liaison double, une liaison triple, et un groupe amino, lorsque p est 0,
groupe III: L₁ et L₂ sont chacun indépendamment un groupe hydrocarbure divalent ayant 1 à 20 atomes de carbone, lorsque p est 1,
groupe IV: L₁ est un groupe hydrocarbure divalent ayant 1 à 20 atomes de carbone, à l'exclusion un groupe méthylène, et dans lequel L₂ est un groupe hydrocarbure divalent ayant 1 à 20 atomes de carbone contenant une liaison éther, lorsque p est 1,
groupe V : L₂ est un groupe hydrocarbure divalent ayant 1 à 20 atomes de carbone, et dans lequel L₁ est un groupe hydrocarbure divalent ayant 1 à 20 atomes de carbone contenant une liaison éther, lorsque p est 1,
et groupe VI : L₁ et L₂ sont chacun indépendamment un groupe hydrocarbure divalent ayant 1 à 20 atomes de carbone et contenant au moins un d'un groupe consistant en une liaison éther, une liaison thioéther, une liaison double, une liaison triple, et un groupe amino, lorsque p est 1, dans laquelle L₁ exclut une liaison éther lorsque L₂ contient une liaison éther, et dans laquelle L₂ exclut une liaison éther lorsque L₁ contient une liaison éther,
dans laquelle ledit polyéthylène glycol ramifié monofonctionnel peut être obtenu par un processus dans lequel 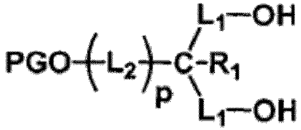
est un initiateur de départ pour une première polymérisation, et dans laquelle 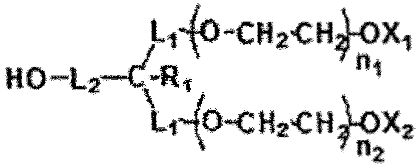
est un intermédiaire pour une deuxième polymérisation, et
PG est un groupe protecteur d'un groupe hydroxyle.

2. PEG ramifié monofonctionnel selon la revendication 1, dans lequel X₁ et X₂ sont chacun indépendamment un groupe choisi dans le groupe consistant en un méthyle, un éthyle, un propyle, un propényle, un propinyle, un isopropyle, un butyle, un butyle tertiaire, un pentyle, un heptyle, un 2-éthylhexyle, un octyle, un nonyle, un décyle, un undécyle, un dodécyle, un tridécyle, un tétradécyle, un pentadécyle, un hexadécyle, un heptadécyle, un octodécyle, un nonadécyle, un eicosyle, un benzyle et un butylphényle, et X₁ et X₂ sont identiques ou différents l'un de l'autre dans une molécule.

3. PEG ramifié monofonctionnel selon la revendication 1, dans lequel R est choisi dans les groupes suivants : 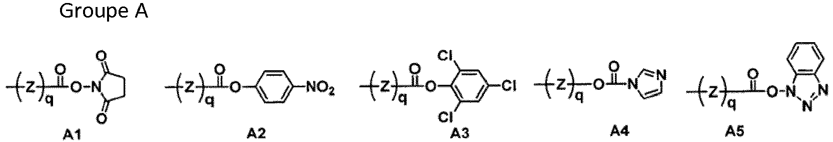 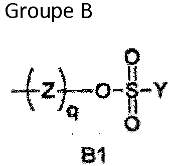 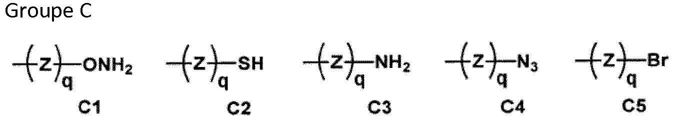 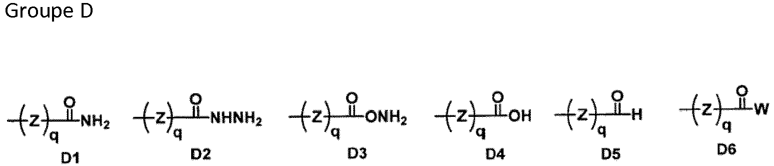 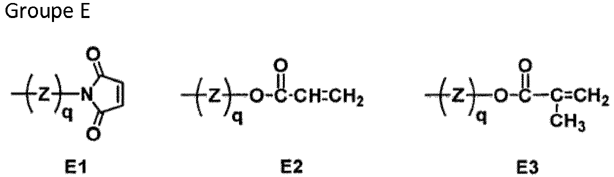 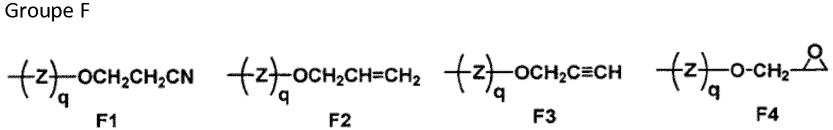 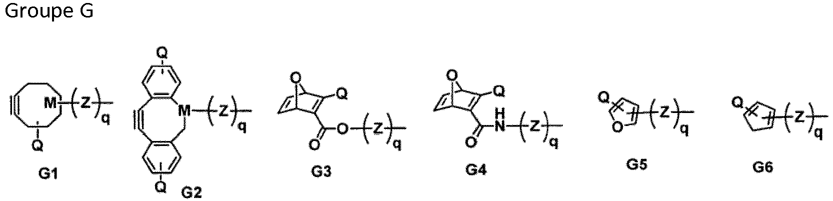 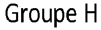 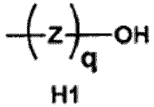
dans lequel Z est un groupe alkylène ou un groupe alkylène contenant au moins un groupe choisi dans un groupe consistant en une liaison amide, une liaison éther, une liaison double, une liaison triple et un groupe amino secondaire,
dans lequel q est 0 ou 1,
dans lequel Y est un groupe hydrocarbure ayant 1 à 10 atomes de carbone ou un groupe hydrocarbure ayant 1 à 10 atomes de carbone qui contient un atome de fluor,
dans lequel Q est un atome d'hydrogène ou un groupe favorisant un effet inductif, un effet de conjugaison ou les deux d'électrons d'une liaison insaturée,
dans lequel M est un atome de carbone ou un atome d'azote sur le cycle, et
dans lequel W est un atome d'halogène.

4. PEG ramifié monofonctionnel selon la revendication 3, dans lequel Z est choisi dans un groupe consistant en : un groupe méthylène, un groupe 1,2-éthy!ène, un groupe 1,3-propylène, un groupe 1,2-propylène, un groupe isopropylène, un groupe butylène, un groupe pentylène et un groupe hexylène.

5. PEG ramifié monofonctionnel selon la revendication 1, dans lequel chacun de n₁ et n₂ est indépendamment une valeur choisie de 10 à 800, de préférence de 25 à 800, et de manière davantage préférée, de 50 à 500.

6. PEG ramifié monofonctionnel selon la revendication 1, dans lequel n₃ est une valeur choisie de 11 à 800, de préférence de 11 à 500, et de manière davantage préférée de 11 à 200.

7. PEG ramifié monofonctionnel selon la revendication 1, dans lequel la substance bio-apparentée est choisie dans un groupe consistant en un polypeptide, une protéine, une enzyme, un médicament à petites molécules, un colorant, un liposome, un nucléoside, un nucléotide, un oligonucléotide, un polynucléotide, un acide nucléique, un polysaccharide, un stéroïde, un lipide, un phospholipide, un glycolipide, une glycoprotéine, une cellule et un virus.

8. PEG ramifié monofonctionnel selon la revendication 1, dans lequel le groupe réactif d'une substance bio-apparentée est choisi dans l'un d'un groupe consistant en un groupe amino, un groupe mercapto, une liaison insaturée, un groupe carboxyle et un groupe hydroxyle.

9. Procédé de production du PEG ramifié monofonctionnel selon l'une quelconque des revendications 1 à 8, comportant les étapes suivantes :
étape (a) : dans un système de co-initiateur consistant en un initiateur à petites molécules (4) et une base, de l'oxyde d'éthylène est polymérisé avec les deux groupes hydroxyle géométriquement symétriques de l'initiateur (4) pour générer deux chaînes ramifiées ; ensuite, les extrémités terminales des deux chaînes ramifiées nouvellement formées sont déprotonées pour obtenir un intermédiaire (5),
étape (b) : les terminaisons actives initiatrices des deux chaînes ramifiées de l'intermédiaire (5) sont alkyl-éthérifiées pour obtenir un intermédiaire (6) ;
étape (c) : le groupe hydroxyle terminal sur l'axe de symétrie de l'intermédiaire (6) est déprotégé pour obtenir un intermédiaire (7) ;
étape (d) : de l'oxyde d'éthylène est polymérisé avec le groupe hydroxyle terminal déprotégé sur l'axe de symétrie de l'intermédiaire (7) pour générer une chaîne principale, qui est ensuite protonée pour obtenir un intermédiaire (3) avec un groupe hydroxyle terminal ;
étape (e) : la terminaison de la chaîne principale de l'intermédiaire (3) est fonctionnalisée, et ainsi le polyéthylène glycol ramifié monofonctionnel de formule générale (1) peut être obtenu ; 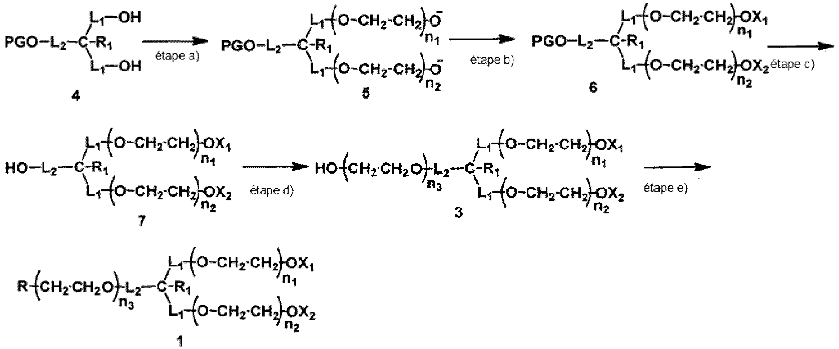
dans lequel PG représente un groupe protecteur d'un groupe hydroxyle.

10. Substance bio-apparentée modifiée par le PEG ramifié monofonctionnel selon l'une quelconque des revendications 1 à 8, et ayant une structure chimique présentée dans la formule générale (2) : 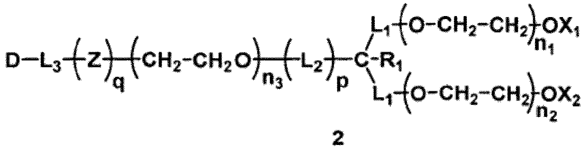
dans laquelle D est une substance bio-apparentée, q est 0 ou 1, et
dans laquelle Z est un groupe de liaison par lequel un groupe fonctionnel capable de réagir avec la substance bio-apparentée est lié à la chaîne principale de PEG, et peut réagir avec la substance bio-apparentée pour former un groupe de résidu L₃.

11. Substance bio-apparentée modifiée par le PEG selon la revendication 10, dans laquelle D est choisi dans un groupe consistant en un polypeptide, une protéine, une enzyme, un médicament à petites molécules, un colorant, un liposome, un nucléoside, un nucléotide, un oligonucléotide, un polynucléotide, un acide nucléique, un polysaccharide, un stéroïde, un lipide, un phospholipide, un glycolipide, une glycoprotéine, une cellule et un virus.

12. Substance bio-apparentée modifiée par le PEG selon la revendication 11, dans laquelle D est choisi dans un groupe consistant en un polypeptide, une protéine, une enzyme, un colorant, un liposome, un nucléoside, un nucléotide, un oligonucléotide, un polynucléotide, un acide nucléique, un polysaccharide, un stéroïde, un lipide, un phospholipide, un glycolipide, une glycoprotéine, une cellule et un virus, de préférence est choisi dans un groupe consistant en un polypeptide, une protéine, une enzyme, un nucléoside, un nucléotide, un oligonucléotide, un polynucléotide et un acide nucléique, et de manière davantage préférée est choisi dans un groupe consistant en un interféron, un lysozyme et un oligodésoxynucléotide anti-sens.

13. Substance bio-apparentée modifiée par le PEG selon la revendication 10, dans laquelle L₃ est choisi dans un groupe consistant en une liaison triazole, une liaison isoxazole, une liaison éther, une liaison amide, une liaison imide, une liaison imino, un groupe amino secondaire, un groupe amino tertiaire, une liaison thioester, une liaison disulfure, une liaison uréthane, une liaison thiocarbonate, une liaison sulfonate, une liaison sulfamide, une liaison carbamate, un groupe tyrosine, un groupe cystéine, un groupe histidine et la combinaison de ceux-ci.

14. Composé de la formule (3) suivante 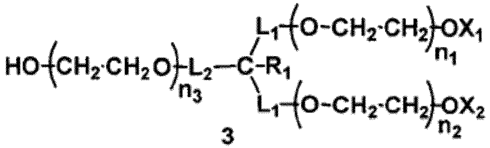 dans laquelle X₁, X₂, n₁, n₂, n₃, L₁, L₂ et R₁ sont les mêmes que ceux définis dans la formule générale (1) de la revendication 1.

15. Procédé de production du composé intermédiaire ayant la formule (3) ci-dessous, qui comporte les étapes suivantes :
étape (a) : dans un système de co-initiateur consistant en un initiateur à petites molécules (4) et une base, de l'oxyde d'éthylène est polymérisé avec les deux groupes hydroxyle géométriquement symétriques de l'initiateur (4) pour générer deux chaînes ramifiées ; ensuite, les extrémités terminales des deux chaînes ramifiées nouvellement formées sont déprotonées pour obtenir un intermédiaire (5),
étape (b) : les terminaisons actives initiatrices des deux chaînes ramifiées de l'intermédiaire (5) sont alkyl-éthérifiées pour obtenir un intermédiaire (6) ;
étape (c) : le groupe hydroxyle terminal sur l'axe de symétrie de l'intermédiaire (6) est déprotégé pour obtenir un intermédiaire (7) ;
étape (d) : l'oxyde d'éthylène est polymérisé avec le groupe hydroxyle terminal déprotégé sur l'axe de symétrie de l'intermédiaire (7) pour générer une chaîne principale, qui est ensuite protonée pour obtenir un intermédiaire (3) avec un groupe hydroxyle terminal ; 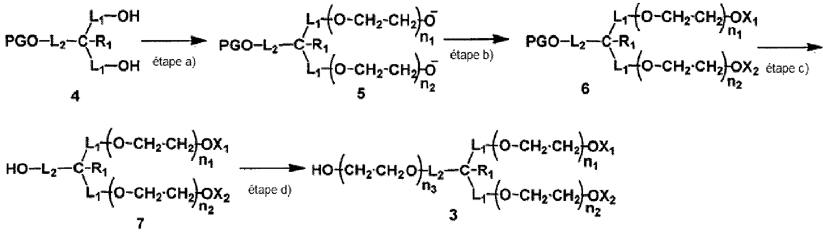 dans lequel PG représente un groupe protecteur d'un groupe hydroxyle.
